# EUROPEAN PATENT SPECIFICATION

(11) **EP 1 731 531 B1**
(45) Date of publication and mention of the grant of the patent: **30.05.2012**
(21) Application number: 06001900.7
(22) Date of filing: 09.08.2000
(51) Int. Cl.: C07K 16/30, C07K 14/54, C07K 14/55, C07K 19/00

(54) **Multiple cytokine-antibody complexes**
Mehrere Zytokin-Antikörper Komplexe
Complexes contenant de multiples cytokines et un anticorps

(30) Priority: 09.08.1999 US 147924 P
(43) Date of publication of application: 13.12.2006
(62) Divisional of application: 00953896.8
(73) Proprietor: Merck Patent GmbH, 64293 Darmstadt (DE)
(72) Inventor: Gillies, Stephen D., Carlisle, MA 01741 (US); LO, Kin Ming, Lexington, MA 02420 (US)
(74) Representative: Benz, Jürgen

(56) References cited:
- WO-A-92/08495
- WO-A1-92/04455
- YE J ET AL: "Cellular and molecular mechanisms of IFN-gamma production induced by IL-2 and IL-12 in a human NK cell line." JOURNAL OF LEUKOCYTE BIOLOGY AUG 1995 LNKD- PUBMED:7643015, vol. 58, no. 2, August 1995 (1995-08), pages 225-233, XP002595761 ISSN: 0741-5400
- S GILLIES ET AL: "Antibody-IL-12 fusion proteins are effective in SCID mouse models of prostate and colon carcinoma metastases" JOURNAL OF IMMUNOLOGY,US,THE WILLIAMS AND WILKINS CO. BALTIMORE, vol. 160, no. 12, 15 June 1998 (1998-06-15), pages 6195-6203, XP002106576 ISSN: 0022-1767
- DECESARE S L ET AL: "Interleukin-12-mediated tumoricidal activity of patient lymphocytes in an autologous in vitro ovarian cancer assay system." GYNECOLOGIC ONCOLOGY APR 1995 LNKD- PUBMED:7705706, vol. 57, no. 1, April 1995 (1995-04), pages 86-95, XP002595762 ISSN: 0090-8258
- KIM J J ET AL: "Cytokine molecular adjuvants modulate immune responses induced by DNA vaccine constructs for HIV-1 and SIV." JOURNAL OF INTERFERON AND CYTOKINE RESEARCH, (1999 JAN) 19 (1) 77-84., January 1999 (1999-01), XP000971666
- REISFELD R A ET AL: "Recombinant antibody fusion proteins for cancer immunotherapy" CURRENT TOPICS IN MICROBIOLOGY AND IMMUNOLOGY, vol. 213, no. pt 3, 1996, pages 27-53, XP002107034
- LEBERTHON B ET AL: "ENHANCED TUMOR UPTAKE OF MACROMOLECULES INDUCED BY A NOVEL VASOACTIVE INTERLEUKIN 2 IMMUNOCONJUGATE" CANCER RESEARCH,US,AMERICAN ASSOCIATION FOR CANCER RESEARCH, BALTIMORE, MD, vol. 51, 15 May 1991 (1991-05-15), pages 2694-2698, XP002069099 ISSN: 0008-5472
- HARVILL E T ET AL: "In vivo properties of an IgG3-IL-2 fusion protein. A general strategy for immune potentiation" JOURNAL OF IMMUNOLOGY,US,THE WILLIAMS AND WILKINS CO. BALTIMORE, 1996, pages 3165-3170, XP002107033 ISSN: 0022-1767

### SEQUENCE LISTING

<110> Gillies, Stephen
   Lo, Kin Ming
<120> Multiple Cytokine Protein Complexes
<130> LEX-010PC
<140>
   <141>
<150> 60/147,924
   <151> 1999-08-09
<160> 32
<170> PatentIn Ver. 2.0
<210> 1
   <211> 582
   <212> DNA
   <213> Mus musculus
<220>
   <223> Description of Artificial Sequence: murine p35 coding sequence for mature protein
<400> 1
   agggtcattc cagtctctgg acctgccagg tgtcttagcc agtcccgaaa cctgctgaag 60
   accacagatg acatggtgaa gacggccaga gaaaaactga aacattattc ctgcactgct 120
   gaagacatcg atcatgaaga catcacacgg gaccaaacca gcacattgaa gacctgttta 180
   ccactggaac tacacaagaa cgagagttgc ctggctacta gagagacttc ttccacaaca 240
   agagggagct gcctgccccc acagaagacg tctttgatga tgaccctgtg ccttggtagc 300
   atctatgagg acttgaagat gtaccagaca gagttccagg ccatcaacgc agcacttcag 360
   aatcacaacc atcagcagat cattctagac aagggcatgc tggtggccat cgatgagctg 420
   atgcagtctc tgaatcataa tggcgagact ctgcgccaga aacctcctgt gggagaagca 480
   gacccttaca gagtgaaaat gaagctctgc atcctgcttc acgccttcag cacccgcgtc 540
   gtgaccatca acagggtgat gggctatctg agctccgcct ga                    582
<210> 2
   <211> 1472
   <212> DNA
   <213> Artificial Sequence
<220>
   <223> Description of Artificial Sequence: murine p40-IL-2 fusion protein coding sequence
<400> 2
   atgtgtcctc agaagctaac atgtgtcctc agaagctaac catctcctgg tttgccatcg 60
   ttttgctggt gtctccactc atggccatgt gggagctgga gaaagacgtt tatgttgtag 120
   aggtggactg gactcccgat gcccctggag aaacagtgaa cctcacctgt gacacgcctg 180
   aagaagatga catcacctgg acctcagacc agagacatgg agtcataggc tctggaaaga 240
   ccctgaccat cactgtcaaa gagtttctag atgctggcca gtacacctgc cacaaaggag 300
   gcgagactct gagccactca catctgctgc tccacaagaa ggaaaatgga atttggtcca 360
   ctgaaatttt aaaaaatttc aaaaacaaga ctttcctgaa gtgtgaagca ccaaattact 420
   ccggacggtt cacgtgctca tggctggtgc aaagaaacat ggacttgaag ttcaacatca 480
   agagcagtag cagttcccct gactctcggg cagtgacatg tggaatggcg tctctgtctg 540
   cagagaaggt cacactggac caaagggact atgagaagta ttcagtgtcc tgccaggagg 600
   atgtcacctg cccaactgcc gaggagaccc tgcccattga actggcgttg gaagcacggc 660
   agcagaataa atatgagaac tacagcacca gcttcttcat cagggacatc atcaaaccag 720
   acccgcccaa gaacttgcag atgaagcctt tgaagaactc acaggtggag gtcagctggg 780
   agtaccctga ctcctggagc actccccatt cctacttctc cctcaagttc tttgttcgaa 840
   tccagcgcaa gaaagaaaag atgaaggaga cagaggaggg gtgtaaccag aaaggtgcgt 900
   tcctcgtaga gaagacatct accgaagtcc aatgcaaagg cgggaatgtc tgcgtgcaag 960
   ctcaggatcg ctattacaat tcctcatgca gcaagtgggc atgtgttccc tgcagggtcc 1020
   gatccccggg taaagcaccc acttcaagct ctacagcgga agcacagcag cagcagcagc 1080
   agcagcagca gcagcagcag cacctggagc agctgttgat ggacctacag gagctcctga 1140
   gcaggatgga gaattacagg aacctgaaac tccccaggat gctcaccttc aaattttact 1200
   tgcccaagca ggccacagaa ttgaaagatc ttcagtgcct agaagatgaa cttggacctc 1260
   tgcggcatgt tctggatttg actcaaagca aaagctttca attggaagat gctgagaatt 1320
   tcatcagcaa tatcagagta actgttgtaa aactaaaggg ctctgacaac acatttgagt 1380
   gccaattcga tgatgagtca gcaactgtgg tggactttct gaggagatgg atagccttct 1440
   gtcaaagcat catctcaaca agccctcaat aa                               1472
<210> 3
   <211> 1409
   <212> DNA
   <213> Artificial Sequence
<220>
   <223> Description of Artificial Sequence: murine p40-GM-CSF fusion protein coding sequence
<400> 3
   atgtgtcctc agaagctaac atgtgtcctc agaagctaac catctcctgg tttgccatcg 60
   ttttgctggt gtctccactc atggccatgt gggagctgga gaaagacgtt tatgttgtag 120
   aggtggactg gactcccgat gcccctggag aaacagtgaa cctcacctgt gacacgcctg 180
   aagaagatga catcacctgg acctcagacc agagacatgg agtcataggc tctggaaaga 240
   ccctgaccat cactgtcaaa gagtttctag atgctggcca gtacacctgc cacaaaggag 300
   gcgagactct gagccactca catctgctgc tccacaagaa ggaaaatgga atttggtcca 360
   ctgaaatttt aaaaaatttc aaaaacaaga ctttcctgaa gtgtgaagca ccaaattact 420
   ccggacggtt cacgtgctca tggctggtgc aaagaaacat ggacttgaag ttcaacatca 480
   agagcagtag cagttcccct gactctcggg cagtgacatg tggaatggcg tctctgtctg 540
   cagagaaggt cacactggac caaagggact atgagaagta ttcagtgtcc tgccaggagg 600
   atgtcacctg cccaactgcc gaggagaccc tgcccattga actggcgttg gaagcacggc 660
   agcagaataa atatgagaac tacagcacca gcttcttcat cagggacatc atcaaaccag 720
   acccgcccaa gaacttgcag atgaagcctt tgaagaactc acaggtggag gtcagctggg 780
   agtaccctga ctcctggagc actccccatt cctacttctc cctcaagttc tttgttcgaa 840
   tccagcgcaa gaaagaaaag atgaaggaga cagaggaggg gtgtaaccag aaaggtgcgt 900
   tcctcgtaga gaagacatct accgaagtcc aatgcaaagg cgggaatgtc tgcgtgcaag 960
   ctcaggatcg ctattacaat tcctcatgca gcaagtgggc atgtgttccc tgcagggtcc 1020
   gatccccggg aaaagcaccc gcccgctcac ccataattgt tacccggcct tggaagcatg 1080
   tagaggccat caaagaagcc ctaaacctcc tggatgacat gcctgtcacg ttgaatgaag 1140
   aggtagaagt cgtctctaac gagttctcct tcaagaagct aacatgtgtg cagacccgcc 1200
   tgaagatatt cgagcagggt ctacggggca atttcaccaa actcaagggc gccttgaaca 1260
   tgacagccag ctactaccag acatactgcc ccccaactcc ggaaacggac tgtgaaacac 1320
   aagttaccac ctatgcggat ttcatagaca gccttaaaac ctttctgact gatatcccct 1380
   ttgaatgcaa aaaaccaagc caaaaatga                                   1409
<210> 4
   <211> 1389
   <212> DNA
   <213> Artificial Sequence
<220>
   <223> Description of Artificial Sequence: human p40-IL-2 fusion protein coding sequence
<400> 4
   atgtgtcacc agcagttggt catctcttgg ttttccctgg tttttctggc atctcccctc 60
   gtggccatat gggaactgaa gaaagatgtt tatgtcgtag aattggattg gtatccggat 120
   gcccctggag aaatggtggt cctcacctgt gacacccctg aagaagatgg tatcacctgg 180
   accttggacc agagcagtga ggtcttaggc tctggcaaaa ccctgaccat ccaagtcaaa 240
   gagtttggag atgctggcca gtacacctgt cacaaaggag gcgaggttct aagccattcg 300
   ctcctgctgc ttcacaaaaa ggaagatgga atttggtcca ctgatatttt aaaggaccag 360
   aaagaaccca aaaataagac ctttctaaga tgcgaggcca agaattattc tggacgtttc 420
   acctgctggt ggctgacgac aatcagtact gatttgacat tcagtgtcaa aagcagcaga 480
   ggctcttctg acccccaagg ggtgacgtgc ggagctgcta cactctctgc agagagagtc 540
   agaggggaca acaaggagta tgagtactca gtggagtgcc aggaggacag tgcctgccca 600
   gctgctgagg agagtctgcc cattgaggtc atggtggatg ccgttcacaa gctcaagtat 660
   gaaaactaca ccagcagctt cttcatcagg gacatcatca aacctgaccc acccaagaac 720
   ttgcagctga agccattaaa gaattctcgg caggtggagg tcagctggga gtaccctgac 780
   acctggagta ctccacattc ctacttctcc ctgacattct gcgttcaggt ccagggcaag 840
   agcaagagag aaaagaaaga tagagtcttc acggacaaga cctcagccac ggtcatctgc 900
   cgcaaaaatg ccagcattag cgtgcgggcc caggaccgct actatagctc atcttggagc 960
   gaatgggcat ctgtgccctg cagtgcacct acttcaagtt ctacaaagaa aacacagcta 1020
   caactggagc atttactgct ggatttacag atgattttga atggaattaa taattacaag 1080
   aatcccaaac tcaccaggat gctcacattt aagttttaca tgcccaagaa ggccacagaa 1140
   ctgaaacatc ttcagtgtct agaagaagaa ctcaaacctc tggaggaagt gctaaattta 1200
   gctcaaagca aaaactttca cttaagaccc agggacttaa tcagcaatat caacgtaata 1260
   gttctggaac taaagggatc tgaaacaaca ttcatgtgtg aatatgctga tgagacagca 1320
   accattgtag aatttctgaa cagatggatt accttttgtc aaagcatcat ctcaacacta 1380
   acttgataa 1389
<210> 5
   <211> 1278
   <212> DNA
   <213> Artificial Sequence
<220>
   <223> Description of Artificial Sequence: murine Fc-p35 fusion protein coding sequence
<400> 5
   gagcccagag ggcccacaat caagccctgt cctccatgca aatgcccagc acctaacctc 60
   ttgggtggac catccgtctt catcttccct ccaaagatca aggatgtact catgatctcc 120
   ctgagcccca tagtcacatg tgtggtggtg gatgtgagcg aggatgaccc agatgtccag 180
   atcagctggt ttgtgaacaa cgtggaagta cacacagctc agacacaaac ccatagagag 240
   gattacaaca gtactctccg ggtggtcagt gccctcccca tccagcacca ggactggatg 300
   agtggcaagg agttcaaatg caaggtcaac aacaaagacc tcccagcgcc catcgagaga 360
   accatctcaa aacccaaagg gtcagtaaga gctccacagg tatatgtctt gcctccacca 420
   gaagaagaga tgactaagaa acaggtcact ctgacctgca tggtcacaga cttcatgcct 480
   gaagacattt acgtggagtg gaccaacaac gggaaaacag agctaaacta caagaacact 540
   gaaccagtcc tggactctga tggttcttac ttcatgtaca gcaagctgag agtggaaaag 600
   aagaactggg tggaaagaaa tagctactcc tgttcagtgg tccacgaggg tctgcacaat 660
   caccacacga ctaagagctt ctcccggacc ccgggtaggg tcattccagt ctctggacct 720
   gccaggtgtc ttagccagtc ccgaaacctg ctgaagacca cagatgacat ggtgaagacg 780
   gccagagaaa aactgaaaca ttattcctgc actgctgaag acatcgatca tgaagacatc 840
   acacgggacc aaaccagcac attgaagacc tgtttaccac tggaactaca caagaacgag 900
   agttgcctgg ctactagaga gacttcttcc acaacaagag ggagctgcct gcccccacag 960
   aagacgtctt tgatgatgac cctgtgcctt ggtagcatct atgaggactt gaagatgtac 1020
   cagacagagt tccaggccat caacgcagca cttcagaatc acaaccatca gcagatcatt 1080
   ctagacaagg gcatgctggt ggccatcgat gagctgatgc agtctctgaa tcataatggc 1140
   gagactctgc gccagaaacc tcctgtggga gaagcagacc cttacagagt gaaaatgaag 1200
   ctctgcatcc tgcttcacgc cttcagcacc cgcgtcgtga ccatcaacag ggtgatgggc 1260
   tatctgagct ccgcctga                                              1278
<210> 6
   <211> 1287
   <212> DNA
   <213> Artificial Sequence
<220>
   <223> Description of Artificial Sequence: human Fc-p35 fusion protein coding sequence
<400> 6
   gagcccaaat cttgtgacaa aactcacaca tgcccaccgt gcccagcacc tgaactcctg 60
   gggggaccgt cagtcttcct cttcccccca aaacccaagg acaccctcat gatctcccgg 120
   acccctgagg tcacatgcgt ggtggtggac gtgagccacg aagaccctga ggtcaagttc 180
   aactggtacg tggacggcgt ggaggtgcat aatgccaaga caaagccgcg ggaggagcag 240
   tacaacagca cgtaccgtgt ggtcagcgtc ctcaccgtcc tgcaccagga ctggctgaat 300
   ggcaaggagt acaagtgcaa ggtctccaac aaagccctcc cagcccccat cgagaaaacc 360
   atctccaaag ccaaagggca gccccgagaa ccacaggtgt acaccctgcc cccatcacgg 420
   gaggagatga ccaagaacca ggtcagcctg acctgcctgg tcaaaggctt ctatcccagc 480
   gacatcgccg tggagtggga gagcaatggg cagccggaga acaactacaa gaccacgcct 540
   cccgtgctgg actccgacgg ctccttcttc ctctatagca agctcaccgt ggacaagagc 600
   aggtggcagc aggggaacgt cttctcatgc tccgtgatgc atgaggctct gcacaaccac 660
   tacacgcaga agagcctctc cctgtccccg ggaagaaacc tccccgtggc cactccagac 720
   ccaggaatgt tcccatgcct tcaccactcc caaaacctgc tgagggccgt cagcaacatg 780
   ctccagaagg ccagacaaac tctagaattt tacccttgca cttctgaaga gattgatcat 840
   gaagatatca caaaagataa aaccagcaca gtggaggcct gtttaccatt ggaattaacc 900
   aagaatgaga gttgcctaaa ttccagagag acctctttca taactaatgg gagttgcctg 960
   gcctccagaa agacctcttt tatgatggcc ctgtgcctta gtagtattta tgaagacttg 1020
   aagatgtacc aggtggagtt caagaccatg aatgcaaagc ttctgatgga tcctaagagg 1080
   cagatctttc tagatcaaaa catgctggca gttattgatg agctgatgca ggccctgaat 1140
   ttcaacagtg agactgtgcc acaaaaatcc tcccttgaag aaccggattt ttataaaact 1200
   aaaatcaagc tctgcatact tcttcatgct ttcagaattc gggcagtgac tattgacaga 1260
   gtgacgagct atctgaatgc ttcctaa                                    1287
<210> 7
   <211> 32
   <212> DNA
   <213> Artificial Sequence
<220>
   <223> Description of Artificial Sequence: forward primer for construction of murine p40-IL-2 fusion protein
<220>
   <221> misc_feature
   <222> (12)..(14)
   <223> translation initiation codon
<400> 7
   aagctagcac catgtgtcct cagaagctaa cc 32
<210> 8
   <211> 27
   <212> DNA
   <213> Artificial Sequence
<220>
   <223> Description of Artificial Sequence: reverse primer for construction of murine p40-IL-2 fusion protein
<220>
   <221> misc_feature
   <222> Complement((7)..(9))
   <223> translation stop codon
<400> 8
   ctcgagctag gatcggaccc tgcaggg 27
<210> 9
   <211> 31
   <212> DNA
   <213> Artificial Sequence
<220>
   <223> Description of Artificial Sequence: DNA sequence at the junction of murine p40-IL-2 fusion protein
<220>
   <221> misc_feature
   <222> (14)..(16)
   <223> encodes the C-terminal amino acid residue of murine p40
<220>
   <221> misc_feature
   <222> (26)..(28)
   <223> encodes the N-terminal amino acid residue of mature murine IL-2
<400> 9
   ctgcagggtc cgatccccgg gtaaagcacc c 31
<210> 10
   <211> 28
   <212> DNA
   <213> Artificial Sequence
<220>
   <223> Description of Artificial Sequence: DNA sequence at the junction of single-chain murine IL12 and GMCSF
<220>
   <221> misc_feature
   <222> (14)..(16)
   <223> encodes the C-terminal amino acid residue of murine p40
<220>
   <221> misc_feature
   <222> (26)..(28)
   <223> encodes the N-terminal amino acid residue of mature murine GMCSF
<400> 10
   ctgcagggtc cgatccccgg gaaaagca 28
<210> 11
   <211> 2013
   <212> DNA
   <213> Artificial Sequence
<220>
   <223> Description of Artificial Sequence: murine p35-linker-p40-IL-2 fusion protein coding sequence
<400> 11
   agggtcattc cagtctctgg acctgccagg tgtcttagcc agtcccgaaa cctgctgaag 60
   accacagatg acatggtgaa gacggccaga gaaaaactga aacattattc ctgcactgct 120
   gaagacatcg atcatgaaga catcacacgg gaccaaacca gcacattgaa gacctgttta 180
   ccactggaac tacacaagaa cgagagttgc ctggctacta gagagacttc ttccacaaca 240
   agagggagct gcctgccccc acagaagacg tctttgatga tgaccctgtg ccttggtagc 300
   atctatgagg acttgaagat gtaccagaca gagttccagg ccatcaacgc agcacttcag 360
   aatcacaacc atcagcagat cattctagac aagggcatgc tggtggccat cgatgagctg 420
   atgcagtctc tgaatcataa tggcgagact ctgcgccaga aacctcctgt gggagaagca 480
   gacccttaca gagtgaaaat gaagctctgc atcctgcttc acgccttcag cacccgcgtc 540
   gtgaccatca acagggtgat gggctatctg agctccgcgt cgagcggggg cagcgggggc 600
   ggaggcagcg gcgggggcgg atccgccatg tgggtgctgg agaaagacgt ttatgttgta 660
   gaggtggact ggactcccga tgcccctgga gaaacagtga acctcacctg tgacacgcct 720
   gaagaagatg acatcacctg gacctcagac cagagacatg gagtcatagg ctctggaaag 780
   accctgacca tcactgtcaa agagtttcta gatgctggcc agtacacctg ccacaaagga 840
   ggcgagactc tgagccactc acatctgctg ctccacaaga aggaaaatgg aatttggtcc 900
   actgaaattt taaaaaattt caaaaacaag actttcctga agtgtgaagc accaaattac 960
   tccggacggt tcacgtgctc atggctggtg caaagaaaca tggacttgaa gttcaacatc 1020
   aagagcagta gcagttcccc tgactctcgg gcagtgacat gtggaatggc gtctctgtct 1080
   gcagagaagg tcacactgga ccaaagggac tatgagaagt attcagtgtc ctgccaggag 1140
   gatgtcacct gcccaactgc cgaggagacc ctgcccattg aactggcgtt ggaagcacgg 1200
   cagcagaata aatatgagaa ctacagcacc agcttcttca tcagggacat catcaaacca 1260
   gacccgccca agaacttgca gatgaagcct ttgaagaact cacaggtgga ggtcagctgg 1320
   gagtaccctg actcctggag cactccccat tcctacttct ccctcaagtt ctttgttcga 1380
   atccagcgca agaaagaaaa gatgaaggag acagaggagg ggtgtaacca gaaaggtgcg 1440
   ttcctcgtag agaagacatc taccgaagtc caatgcaaag gcgggaatgt ctgcgtgcaa 1500
   gctcaggatc gctattacaa ttcctcatgc agcaagtggg catgtgttcc ctgcagggtc 1560
   cgatccccgg gtaaagcacc cacttcaagc tctacagcgg aagcacagca gcagcagcag 1620
   cagcagcagc agcagcagca gcacctggag cagctgttga tggacctaca ggagctcctg 1680
   agcaggatgg agaattacag gaacctgaaa ctccccagga tgctcacctt caaattttac 1740
   ttgcccaagc aggccacaga attgaaagat cttcagtgcc tagaagatga acttggacct 1800
   ctgcggcatg ttctggattt gactcaaagc aaaagctttc aattggaaga tgctgagaat 1860
   ttcatcagca atatcagagt aactgttgta aaactaaagg gctctgacaa cacatttgag 1920
   tgccaattcg atgatgagtc agcaactgtg gtggactttc tgaggagatg gatagccttc 1980
   tgtcaaagca tcatctcaac aagccctcaa taa                              2013
<210> 12
   <211> 1569
   <212> DNA
   <213> Artificial Sequence
<220>
   <223> Description of Artificial Sequence: murine p35-linker-p40 fusion protein coding sequence
<400> 12
   agggtcattc cagtctctgg acctgccagg tgtcttagcc agtcccgaaa cctgctgaag 60
   accacagatg acatggtgaa gacggccaga gaaaaactga aacattattc ctgcactgct 120
   gaagacatcg atcatgaaga catcacacgg gaccaaacca gcacattgaa gacctgttta 180
   ccactggaac tacacaagaa cgagagttgc ctggctacta gagagacttc ttccacaaca 240
   agagggagct gcctgccccc acagaagacg tctttgatga tgaccctgtg ccttggtagc 300
   atctatgagg acttgaagat gtaccagaca gagttccagg ccatcaacgc agcacttcag 360
   aatcacaacc atcagcagat cattctagac aagggcatgc tggtggccat cgatgagctg 420
   atgcagtctc tgaatcataa tggcgagact ctgcgccaga aacctcctgt gggagaagca 480
   gacccttaca gagtgaaaat gaagctctgc atcctgcttc acgccttcag cacccgcgtc 540
   gtgaccatca acagggtgat gggctatctg agctccgcgt cgagcggggg cagcgggggc 600
   ggaggcagcg gcgggggcgg atccgccatg tgggtgctgg agaaagacgt ttatgttgta 660
   gaggtggact ggactcccga tgcccctgga gaaacagtga acctcacctg tgacacgcct 720
   gaagaagatg acatcacctg gacctcagac cagagacatg gagtcatagg ctctggaaag 780
   accctgacca tcactgtcaa agagtttcta gatgctggcc agtacacctg ccacaaagga 840
   ggcgagactc tgagccactc acatctgctg ctccacaaga aggaaaatgg aatttggtcc 900
   actgaaattt taaaaaattt caaaaacaag actttcctga agtgtgaagc accaaattac 960
   tccggacggt tcacgtgctc atggctggtg caaagaaaca tggacttgaa gttcaacatc 1020
   aagagcagta gcagttcccc tgactctcgg gcagtgacat gtggaatggc gtctctgtct 1080
   gcagagaagg tcacactgga ccaaagggac tatgagaagt attcagtgtc ctgccaggag 1140
   gatgtcacct gcccaactgc cgaggagacc ctgcccattg aactggcgtt ggaagcacgg 1200
   cagcagaata aatatgagaa ctacagcacc agcttcttca tcagggacat catcaaacca 1260
   gacccgccca agaacttgca gatgaagcct ttgaagaact cacaggtgga ggtcagctgg 1320
   gagtaccctg actcctggag cactccccat tcctacttct ccctcaagtt ctttgttcga 1380
   atccagcgca agaaagaaaa gatgaaggag acagaggagg ggtgtaacca gaaaggtgcg 1440
   ttcctcgtag agaagacatc taccgaagtc caatgcaaag gcgggaatgt ctgcgtgcaa 1500
   gctcaggatc gctattacaa ttcctcatgc agcaagtggg catgtgttcc ctgcagggtc 1560
   cgatcctag                                                         1569
<210> 13
   <211> 2709
   <212> DNA
   <213> Artificial Sequence
<220>
   <223> Description of Artificial Sequence: murine Fc-p35-linker-p40-IL-2 fusion protein coding sequence
<400> 13
   gagcccagag ggcccacaat caagccctgt cctccatgca aatgcccagc acctaacctc 60
   ttgggtggac catccgtctt catcttccct ccaaagatca aggatgtact catgatctcc 120
   ctgagcccca tagtcacatg tgtggtggtg gatgtgagcg aggatgaccc agatgtccag 180
   atcagctggt ttgtgaacaa cgtggaagta cacacagctc agacacaaac ccatagagag 240
   gattacaaca gtactctccg ggtggtcagt gccctcccca tccagcacca ggactggatg 300
   agtggcaagg agttcaaatg caaggtcaac aacaaagacc tcccagcgcc catcgagaga 360
   accatctcaa aacccaaagg gtcagtaaga gctccacagg tatatgtctt gcctccacca 420
   gaagaagaga tgactaagaa acaggtcact ctgacctgca tggtcacaga cttcatgcct 480
   gaagacattt acgtggagtg gaccaacaac gggaaaacag agctaaacta caagaacact 540
   gaaccagtcc tggactctga tggttcttac ttcatgtaca gcaagctgag agtggaaaag 600
   aagaactggg tggaaagaaa tagctactcc tgttcagtgg tccacgaggg tctgcacaat 660
   caccacacga ctaagagctt ctcccggacc ccgggtaggg tcattccagt ctctggacct 720
   gccaggtgtc ttagccagtc ccgaaacctg ctgaagacca cagatgacat ggtgaagacg 780
   gccagagaaa aactgaaaca ttattcctgc actgctgaag acatcgatca tgaagacatc 840
   acacgggacc aaaccagcac attgaagacc tgtttaccac tggaactaca caagaacgag 900
   agttgcctgg ctactagaga gacttcttcc acaacaagag ggagctgcct gcccccacag 960
   aagacgtctt tgatgatgac cctgtgcctt ggtagcatct atgaggactt gaagatgtac 1020
   cagacagagt tccaggccat caacgcagca cttcagaatc acaaccatca gcagatcatt 1080
   ctagacaagg gcatgctggt ggccatcgat gagctgatgc agtctctgaa tcataatggc 1140
   gagactctgc gccagaaacc tcctgtggga gaagcagacc cttacagagt gaaaatgaag 1200
   ctctgcatcc tgcttcacgc cttcagcacc cgcgtcgtga ccatcaacag ggtgatgggc 1260
   tatctgagct ccgcgtcgag cgggggcagc gggggcggag gcagcggcgg gggcggatcc 1320
   gccatgtggg tgctggagaa agacgtttat gttgtagagg tggactggac tcccgatgcc 1380
   cctggagaaa cagtgaacct cacctgtgac acgcctgaag aagatgacat cacctggacc 1440
   tcagaccaga gacatggagt cataggctct ggaaagaccc tgaccatcac tgtcaaagag 1500
   tttctagatg ctggccagta cacctgccac aaaggaggcg agactctgag ccactcacat 1560
   ctgctgctcc acaagaagga aaatggaatt tggtccactg aaattttaaa aaatttcaaa 1620
   aacaagactt tcctgaagtg tgaagcacca aattactccg gacggttcac gtgctcatgg 1680
   ctggtgcaaa gaaacatgga cttgaagttc aacatcaaga gcagtagcag ttcccctgac 1740
   tctcgggcag tgacatgtgg aatggcgtct ctgtctgcag agaaggtcac actggaccaa 1800
   agggactatg agaagtattc agtgtcctgc caggaggatg tcacctgccc aactgccgag 1860
   gagaccctgc ccattgaact ggcgttggaa gcacggcagc agaataaata tgagaactac 1920
   agcaccagct tcttcatcag ggacatcatc aaaccagacc cgcccaagaa cttgcagatg 1980
   aagcctttga agaactcaca ggtggaggtc agctgggagt accctgactc ctggagcact 2040
   ccccattcct acttctccct caagttcttt gttcgaatcc agcgcaagaa agaaaagatg 2100
   aaggagacag aggaggggtg taaccagaaa ggtgcgttcc tcgtagagaa gacatctacc 2160
   gaagtccaat gcaaaggcgg gaatgtctgc gtgcaagctc aggatcgcta ttacaattcc 2220
   tcatgcagca agtgggcatg tgttccctgc agggtccgat ccccgggtaa agcacccact 2280
   tcaagctcta cagcggaagc acagcagcag cagcagcagc agcagcagca gcagcagcac 2340
   ctggagcagc tgttgatgga cctacaggag ctcctgagca ggatggagaa ttacaggaac 2400
   ctgaaactcc ccaggatgct caccttcaaa ttttacttgc ccaagcaggc cacagaattg 2460
   aaagatcttc agtgcctaga agatgaactt ggacctctgc ggcatgttct ggatttgact 2520
   caaagcaaaa gctttcaatt ggaagatgct gagaatttca tcagcaatat cagagtaact 2580
   gttgtaaaac taaagggctc tgacaacaca tttgagtgcc aattcgatga tgagtcagca 2640
   actgtggtgg actttctgag gagatggata gccttctgtc aaagcatcat ctcaacaagc 2700
   cctcaataa                                                         2709
<210> 14
   <211> 33
   <212> DNA
   <213> Artificial Sequence
<220>
   <223> Description of Artificial Sequence: forward primer for PCR amplification of murine p35 subunit of IL-12
<220>
   <221> misc_feature
   <222> (16)..(18)
   <223> translation initiation codon
<400> 14
   aagcttgcta gcagcatgtg tcaatcacgc tac 33
<210> 15
   <211> 30
   <212> DNA
   <213> Artificial Sequence
<220>
   <223> Description of Artificial Sequence: reverse primer for PCR amplification of murine p35 subunit of IL-12
<220>
   <221> misc_feature
   <222> Complement((10)..(12))
   <223> translation stop codon
<400> 15
   ctcgagcttt caggcggagc tcagatagcc 30
<210> 16
   <211> 61
   <212> DNA
   <213> Artificial Sequence
<220>
   <223> Description of Artificial Sequence: coding sequence at the junction between p35 and p40 that comprise the murine single-chain IL-12
<220>
   <221> misc_feature
   <222> (8)..(10)
   <223> encodes the C-terminal amino acid residue of murine p35
<220>
   <221> misc_feature
   <222> (59)..161)
   <223> encodes the N-terminal amino acid residue of mature murine p40
<400> 16
   gagctccgcg tcgagcgggg gcagcggggg cggaggcagc ggcgggggcg gatccgccat 60
   g                                                                 61
<210> 17
   <211> 16
   <212> PRT
   <213> Artificial Sequence
<220>
   <223> Description of Artificial Sequence: Protein sequence at the junction between p35 and p40 that comprise the murine single-chain IL-12
<400> 17
   Ser Ser Gly Gly Ser Gly Gly Gly Gly Ser Gly Gly Gly Gly Ser Ala
   1               5                   10                  15
<210> 18
   <211> 73
   <212> DNA
   <213> Artificial Sequence
<220>
   <223> Description of Artificial Sequence: coding sequence at the junction between murine p40 and the mature N-terminus of KS heavy chain
<220>
   <221> misc_feature
   <222> (14)..(16)
   <223> encodes the C-terminal amino acid residue of murine p40
<220>
   <221> misc_feature
   <222> (71)..(73)
   <223> encodes the N-terminal residue of mature KS heavy chain
<400> 18
   ctgcagggtc cgatccccgg gatccggagg ttcagggggc ggaggtagcg gcggaggggg 60
   ctccttaagc cag                                                    73
<210> 19
   <211> 18
   <212> PRT
   <213> Artificial Sequence
<220>
   <223> Description of Artificial Sequence: protein sequence at the junction between murine p40 and the mature N-terminus of KS heavy chain
<400> 19
   Pro Gly Ser Gly Gly Ser Gly Gly Gly Gly Ser Gly Gly Gly Gly Ser
    1               5                  10                  15
   Leu Ser
<210> 20
   <211> 64
   <212> DNA
   <213> Artificial Sequence
<220>
   <223> Description of Artificial Sequence: coding sequence at the junction between murine p35 and the KS light chain
<220>
   <221> misc_feature
   <222> (8)..(10)
   <223> encodes the C-terminal amino acid residue of murine p35
<220>
   <221> misc_feature
   <222> (62)..(64)
   <223> encodes the N-terminal amino acid residue of the light chain
<400> 20
   gagctccgcg tcgagcgggg gcagcggggg cggaggcagc ggcgggggcg gatccttaag 60
   cgag                                                              64
<210> 21
   <211> 17
   <212> PRT
   <213> Artificial Sequence
<220>
   <223> Description of Artificial Sequence: protein sequence at the junction between murine p35 and the KS light chain
<400> 21
   Ser Ser Gly Gly Ser Gly Gly Gly Gly Ser Gly Gly Gly Gly Ser Leu
    1               5                  10                  15
   Ser
<210> 22
   <211> 27
   <212> DNA
   <213> Artificial Sequence
<220>
   <223> Description of Artificial Sequence: forward primer for the PCR amplification of murine IL-4
<220>
   <221> misc_feature
   <222> (9)..(11)
   <223> translation initiation codon
<400> 22
   tctagaccat gggtctcaac ccccagc 27
<210> 23
   <211> 47
   <212> DNA
   <213> Artificial Sequence
<220>
   <223> Description of Artificial Sequence: reverse primer for the PCR amplification of murine IL-4
<220>
   <221> misc_feature
   <222> Complement((8)..(10))
   <223> encodes the C-terminal amino acid residue of murine IL-4
<400> 23
   cggatcccga gtaatccatt tgcatgatgc tctttaggct ttccagg 47
<210> 24
   <211> 57
   <212> DNA
   <213> Artificial Sequence
<220>
   <223> Description of Artificial Sequence: coding sequence at the junction of murine IL-4 and the mature KS-1/4 light chain
<220>
   <221> misc_feature
   <222> (1)..(3)
   <223> encodes the C-terminal serine residue of murine IL-4
<220>
   <221> misc_feature
   <222> (55)..(57)
   <223> encodes the N-terminal amino acid residue of the mature KS-1/4 light chain
<400> 24
   tcgggatccg gaggttcagg gggcggaggt agcggcggag ggggctcctt aagcgag 57
<210> 25
   <211> 19
   <212> PRT
   <213> Artificial Sequence
<220>
   <223> Description of Artificial Sequence: protein sequence at the junction of murine IL-4 and the mature KS-1/4 light chain
<400> 25
   Ser Gly Ser Gly Gly Ser Gly Gly Gly Gly Ser Gly Gly Gly Gly Ser
    1               5                  10                  15
   Leu Ser Glu
<210> 26
   <211> 27
   <212> DNA
   <213> Artificial Sequence
<220>
   <223> Description of Artificial Sequence: forward primer for the PCR amplification of murine IL-4
<220>
   <221> misc_feature
   <222> (9)..(11)
   <223> translation initiation codon
<400> 26
   tctagaccat gggtctcaac ccccagc 27
<210> 27
   <211> 52
   <212> DNA
   <213> Artificial Sequence
<220>
   <223> Description of Artificial Sequence: reverse primer for the PCR amplification of murine IL-4
<220>
   <221> misc_feature
   <222> Complement((13)..(15))
   <223> encodes the C-terminal amino acid residue of murine IL-4
<400> 27
   cgatatcccg gacgagtaat ccatttgcat gatgctcttt aggctttcca gg 52
<210> 28
   <211> 39
   <212> DNA
   <213> Artificial Sequence
<220>
   <223> Description of Artificial Sequence: coding sequence at the junction between murine IL-4 and murine GM-CSF
<220>
   <221> misc_feature
   <222> (1)..(12)
   <223> encodes the C-terminal sequence of muIL4
<220>
   <221> misc_feature
   <222> (28)..(39)
   <223> encodes the N-terminal sequence of muGM-CSF
<400> 28
   atggattact cgtccgggat gggaaaagca cccgcccgc 39
<210> 29
   <211> 32
   <212> DNA
   <213> Artificial Sequence
<220>
   <223> Description of Artificial Sequence: forward primer for the PCR amplification of murine lymphotactin
<220>
   <221> misc_feature
   <222> (13)..(15)
   <223> translation initiation codon
<400> 29
   tctagagcca ccatgagact tctcctcctg ac 32
<210> 30
   <211> 26
   <212> DNA
   <213> Artificial Sequence
<220>
   <223> Description of Artificial Sequence: reverse primer for the PCR amplification of murine lymphotactin
<220>
   <221> misc_feature
   <222> Complement((7)..(9))
   <223> encodes the C-terminal amino acid residue of murine lymphotactin
<400> 30
   ggatccccca gtcagggtta ctgctg 26
<210> 31
   <211> 57
   <212> DNA
   <213> Artificial Sequence
<220>
   <223> Description of Artificial Sequence: coding sequence at the junction between murine lymphotactin and KS-IL2 heavy chain
<220>
   <221> misc_feature
   <222> (1)..(3)
   <223> encodes the C-terminal amino acid residue of murine lymphotactin
<220>
   <221> misc_feature
   <222> (55)..(57)
   <223> encodes the N-terminal amino acid residue of the KS-IL2 heavy chain
<400> 31
   cccggatccg gaggttcagg gggcggaggt agcggcggag ggggctcctt aagccag 57
<210> 32
   <211> 17
   <212> PRT
   <213> Artificial Sequence
<220>
   <223> Description of Artificial Sequence: protein sequence at the junction between murine lymphotactin and KS-IL2 heavy chain
<400> 32
   Gly Ser Gly Gly Ser Gly Gly Gly Gly Ser Gly Gly Gly Gly Ser Leu
    1               5                  10                  15
   Ser

## Description

### Field of the Invention

The present invention relates to methods for the construction and expression of multiple cytokine protein complexes and their compositions. More specifically, the invention relates to fusion proteins composed of multiple cytokines and a targeting component, and methods of using the same for the treatment of diseases such as cancer and viral infection.

### Background of the Invention

The regulatory networks controlling the immune system rely on secreted protein signaling molecules termed cytokines to turn on and off the functions of immune cells as well regulate their proliferation. These responses generally involve multiple cytokines that act in concert to achieve the desired biological effect. Certain cytokines such as interleukin-2 (IL-2) can induce immune cell proliferation by themselves and can activate other functions including secondary cytokine secretion. Another cytokine, inteneukin-12 (IL-12) [reviewed by Trinchieri, 1994, Blood 84:4008-4027], can induce proliferation of certain immune cells and induce another key immune modulator, interferon-γ (IFN-y). This induction of IFN-γ is a key activity of IL-12, although IL-12 has other important activities that are IFN-γ independent. Since IL-12 itself is induced at an early stage in infectious disease situations, it is thought to link the innate and acquired immune systems.

Many *in vitro* studies with both mouse and human immune cells have shown the importance of cytokine combinations in the development of optimal immune responses. For example, most T cells do not express IL-12 receptors (IL-12R) until they have been activated with mitogens or cultured in high concentrations of IL-2 [Desai et al. (1992), J. Immunol. 148:3125-3132]. Once the receptors are expressed, the cells become far more responsive to IL-12. Furthermore, IL-12 induces IFN-γ transcription, but IFN-γ mRNA is degraded shortly thereafter. In the presence of IL-2, the mRNA is stabilized, resulting in a dramatic increase in the amount of IFN-γ produced [Chan et al. (1992) J. Immunol. 148:92-98]. In other studies, it was found that the cytokine combinations IL-3 plus IL-11 or IL-3 plus Steel Factor had a synergistic effect with IL-12 on the proliferation of early hematopoietic progenitor cells [Trinchieri, 1994; cited above]. The combination of interleukin-4 and GM-CSF is particularly useful in stimulating dendritic cells (Palucka et al. [1998] J. Immunology 160:4587-4595). For stimulation of the cell-mediated immune response, it is also useful to combine Ih-12 with IL-18, a recently discovered Th1-promoting cytokine with some activities that are complementary to IL-12 (Hashimoto et al. [1999] J. Immunology 163:583-589; Barbulescu et al. [1998] J. Immunology 160:3642-3647). In addition, IL-2 and interferon-γ are synergistic in certain circumstances [Palladino, M. A., U.S. Patent No. 5,082,658].

In many of these synergy studies it was found that the relative level of each cytokine was very important. Whereas the addition of IL-12 in the presence of suboptimal amounts of IL-2 led to synergy in the induction of proliferation, cytolytic activity and IFÑ□ induction, combinations of IL-2 and IL-12 using a high dose of one cytokine were found to be antagonistic [Perussia et al., J. Immunol. 149:3495-3502 (1992); Mehrotra et al., J. Immunol. 151:2444-2452 (1993)]. A similar situation also exists in combinations of IL-12 and IL-7.

Synergy studies between IL-12 and other cytokines for the generation of anti-tumor responses in mice have also shown mixed results. In some models synergy was seen at suboptimal doses of each cytokine and higher doses led to enhanced toxicity, while in other models, combinations of IL-12 and IL-2 showed little or no synergy [see, for example, Nastala et al., J. Immunol. 153:1697-1706. (1994)]. These results may reflect the inherent difficulty of combining two potentially synergistic agents *in vivo,* especially when there is the need to maintain a fixed ratio of activities of two agents with different pharmacological properties, such as different circulating half-life and biodistribution.

In *in vitro* cell culture experiments, it is straightforward to control cytokine levels, but many factors can affect the relative biodistribution and localization of cytokines *in vivo,* thus affecting their immunostimulatory capacity. The most important of these factors is the half-life. The half-life of IL-2 in the circulation after bolus injection is approximately 10 minutes. In striking contrast to these pharmacokinetic properties, the circulating half-life of IL-12 has been reported to be >3 hr in mice [Wysocka et al (1995) Eur. J. Immunol. 25:672] and from 5-10 hr in humans [Lotze et al. (1996) Ann NY Acad Sci 795:440-454].

This difference is thought to be due to the relatively small sizes of both IL-2 and GM-CSF (15-25 kD vs. 75 kD for IL-12), allowing IL-2 and GM-CSF to be cleared by renal filtration. Proteins with a molecular weight of less than about 50 kD are cleared by renal filtration. Almost all cytokines are smaller than 50 kD and undergo similar, rapid clearance by renal filtration. When treatment with two such small, rapidly cleared cytokines is desired, it is sufficient to simply co-administer the cytokines. However, co-administration is not optimal for cytokines with significantly different half-lives.

The systemic administration of cytokines is difficult due to their deleterious side effects. For example, high levels of Interferon-alpha result in significant side effects, including skin, neurologic, immune and endocrine toxicities. It is expected that multiple cytokine fusions might show particularly serious side effects.

To reduce side effects of systemic administration of cytokines, one strategy is to fuse a cytokine to a second molecule with targeting capability. Fusions in which an Fc region is placed at the N-terminus of a another protein (termed 'immunofusins' or 'Fc-X' fusions, where X is a ligand such as Interferon-alpha) have a number of distinctive, advantageous biological properties [Lo et al., U. S. Patent Nos. 5,726,044 and 5,541,087; Lo et al., Protein Engineering 11:495]. In particular, such fusion proteins can still bind to the relevant Fc receptors on cell surfaces. However, when the ligand binds to its receptor on a cell surface, the orientation of the Fc region is altered and the sequences that mediate antibody-dependent cell-mediated cytotoxicity (ADCC) and complement fixation appear to be occluded. As a result, the Fc region in an Fc-X molecule does not mediate ADCC or complement fixation effectively. The cytotoxic effect due to the fusion of an N-terminal cytokine and a C-terminal Fc region is well known. For example, fusion of IL-2 to the N-terminus of an Fc region creates a molecule that is able to bind to cells bearing the IL-2 receptor, fix complement, and lyse the cells as a result [Landolfi, N. F. (1993) U.S. PatentNo. 5,349,053]. In contrast, Fc-IL-2 fusion proteins do not have this property. Thus, Fc-X fusions are expected to have the virtues of increased serum half-life and relative concentration in the liver, without the deleterious effects of ADCC and complement fixation.

It has been demonstrated that many different proteins with short serum half-lives can be fused to an Fc region in an Fc-X configuration, and the resulting fusions have much longer serum half-lives. However, the serum half-lives of two different Fc fusions will not generally be identical. Thus, when delivery of two different X moieties is desired, co-administration of two different Fc-X proteins will not generally be optimal.

Under some circumstances, a better approach is to target the effect of the cytokine to a cell surface antigen by fusing it to an antibody (or fragment derived therefrom) having specificity and affinity for that antigen (Gillies, U.S. Patent No. 5,650,150; Gillies et al., Proc. Natl. Acad. Sci. 89:1428) or by linking a protein antigen and stimulatory cytokine via a peptide linkage in the form of a fusion protein (Hazama et al, Vaccine 11:629). While antibodies themselves can increase the half-life of a fused cytokine, there are still differences between different cytokine fusions with the same antibody [see, for example, Gillies et al., Bioconjugate Chem. 4:230-235 (1993); Gillies et al., J. Immunol. 160:6195-6203] that would make co-localization at a target site difficult. As discussed above, this could lead to an imbalance in cytokine activities and decrease the desired synergistic effects. In addition, the use of two different fusion proteins requires testing each fusion separately for its safety and effectiveness profile, and then further testing as mixtures.

The use of two specific separate cytokines such as IL-12 and IL2 are known to increase synergistically the induction of IFN-gamma and GM-CSF, and thus promotes immunologic responses (Ye et al., 1995, J. Leucocyte Biol., 58, 225-233).

The use of two cytokines fused together is known, for example, from WO 92/04455, which describes in detail fusion proteins of IL3 to a IL3, IL6, EPO, G-CSF and others, which are effective as hematopoiesis stimulators.

The use of Antibody-IL12 fusion proteins that are effective in SCID mouse models of prostate and colon cancer metastases is described by Gillies et al. (J. Immunol., 1998, 160, 6195-6203).

### Summary of the invention:

The present invention provides, specifically, a cytokine-antibody fusion protein having the structure KS-IL12-IL2, wherein KS is the antibody KS 1/4.

Generally, the invention relates to a protein complex containing at least two different cytokines. The cytokines could be in the same polypeptide chain or connected by a covalent bond such as a disulfide bond or a bond formed by chemical crosslinking. Alternatively, the cytokines could be in a stable, non-covalent association. In some preferred embodiments, the protein complex comprises a targeting moiety, such as an antibody or antibody fragment, that targets the complex to a locus in a mammal.

In a preferred embodiment, the invention provides a protein complex combining the bioactivity of a two-chain cytokine, such as IL-12, with that of a second cytokine. The cytokines may be covalently bonded (*e.g*. fused) to each other. The cytokines may also be associated through other moieties. For example, the polypeptide chain containing the second cytokine could include a binding moiety that specifically binds IL-12, such as an antibody to IL-12 or a receptor to IL-12. Alternatively, the binding moiety could interact with a second moiety that is associated with the IL-12. For example, if a polypeptide chain encoding a subunit of IL-12 also includes avidin, the polypeptide containing the second cytokine may include biotin as a targeting moiety. In one preferred embodiment, the second cytokine is IL-2.

The invention provides methods for the production of fusion proteins of IL-12 that maintain both IL-12 activity and that of the second cytokine, while providing a longer, single pharmacokinetic behavior, similar to that of IL-12 itself, that increases the duration of the activity of the second cytokine and maintains the balance of activities of the two cytokines after injection into an animal.

In another embodiment of the invention, the fusion proteins comprise a heterodimeric form of IL-12 in which the p35 and p40 subunits of IL-12 are linked by a disulfide bond and covalently bonded to a second cytokine at either the amino or carboxyl terminus of the p35 or p40 subunit of IL-12 with the general formula IL-12-X or X-IL-12, where X is a second cytokine.

In yet another embodiment, two cytokines are further fused to a protein capable of forming a dimeric or multimeric structure, at either the amino or carboxyl terminus of said protein chain. In a preferred form of this embodiment, one of the fusion protein forms of IL-12 with a second cytokine is further fused to a portion of an immunoglobulin (Ig) chain, such as the Fc region, that is capable of dimerization. Further embodiments include fusion of at least one polypeptide chain of IL-12 at either terminus of a portion of an Ig chain and a second cytokine fused at the other terminus.

In another embodiment, two or more cytokines are fused to a protein with targeting capability by virtue of binding to a specific receptor. For example, an Fc region is capable of binding to Fc receptors, which are abundant in the liver. Fusions of an Fc region with multiple cytokines illustrate the advantages of both dimerization and targeting, but in some circumstances it is useful to construct fusions of multiple cytokines that have only multimerization or targeting capability, but not both capabilities.

In yet another embodiment, a fusion protein comprising multiple cytokines is further fused at either the amino or carboxyl terminus to a member of a class of molecules with diverse targeting capability, such as an antibody or a peptide aptamer with or without a scaffold (Colas et al. [1998] Proc Natl Acad Sci USA. 95:14272-7). A particular embodiment is the fusion of multiple cytokines to at least a portion of an antibody capable of binding an antigen, such as an intact antibody, a single-chain antibody, or a single-chain Fv region. Further embodiments include fusions of at least one polypeptide chain of IL-12 at either terminus of at least a portion of an antibody chain that is capable of binding an antigen, and a second cytokine fused at the other terminus.

The invention also provides nucleic acids that encode fusion proteins comprising two or more cytokines, where one of the cytokines is preferably IL-12 and the fusion protein encoded by the nucleic acid optionally includes other protein moieties. Preferred embodiments include nucleic acids that encode fusions of two or more cytokines to a dimerizing protein, such as an Fc portion of an antibody chain. Another set of preferred embodiments are nucleic acids that encode fusions of two or more cytokines to a protein with targeting capability, such as an antibody.

The invention also provides methods for construction of fusions of two or more cytokines, as well as methods for expression of such fusion proteins.

The invention also provides methods for treatment of diseases and other medical conditions, in which treatment involves the useful combination of the activity of two or more proteins. In one embodiment, at least one of the proteins has a short (*e.g*. less than 20 minutes) or only moderately long (*e.g*. less than 40 minutes) serum half-life. The proteins are fused by genetic engineering or other techniques and administered to a human or animal. In this way, the activities of the two proteins are present in a fixed ratio, and separate administrations on different dosing schedules of the two proteins are not required. In addition, the serum half-life of the fusion protein will generally be more similar to that of the protein component with the longer serum half-life, thus lengthening the effective half-life of the protein or proteins with the shorter serum half-life.

More specifically, the invention provides methods of immune-therapeutic treatment of diseases, such as cancer or infections or other diseases, that might be usefully treated with a two-chain cytokine such as IL-12 in combination with a second cytokine. In a preferred embodiment, IL-12 is fused with IL-2 and administered to an animal or human Such treatments can be used in combination with other disease treatments. In addition, the invention provides methods of vaccination against diverse antigens, which can be used to prevent or treat various diseases.

In yet other embodiments of these methods, two different cytokines are fused to an intact antibody, and are administered to an animal or human. In a preferred form of these methods, the cytokine IL-12 is fused to the antibody moiety along with a second cytokine that is more preferably IL-2.

### Brief Description of the Drawings

The preceding and other objects of the present invention, and the various features thereof, may be more fully understood from the following descriptions, when read together with the accompanying drawings. Throughout the drawings, like numbers refer to like structures.

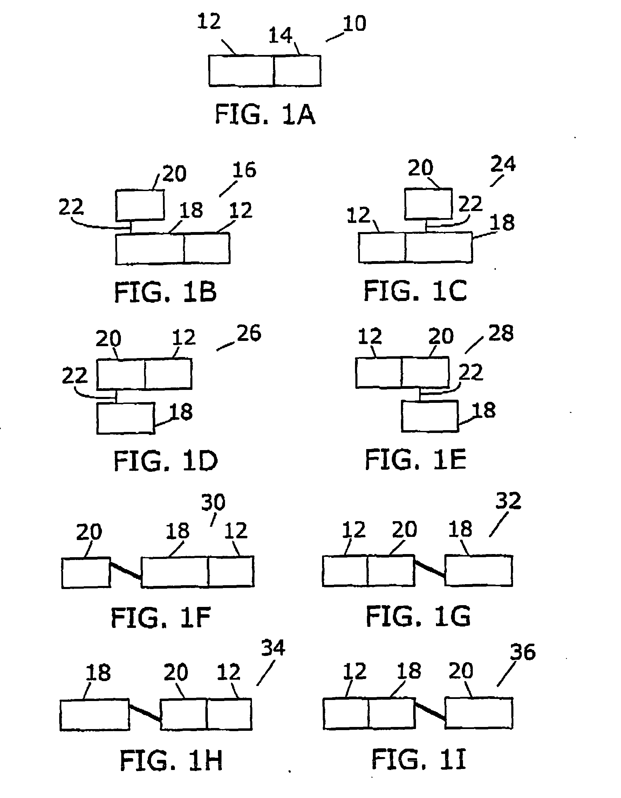

Figure 1A schematically illustrates the fusion of two cytokines in its simplest form: one cytokine is fused to a second cytokine, optionally through a linker. Figures 1B-1I show various ways in which a second cytokine (labeled 'cyt') may be attached to the heterodimeric cytokine IL-12. Specifically, the second cytokine can be fused to the C-terminus of p40 (Figure 1B), the N-terminus of p40 (Figure 1 C), the C-terminus of p35 (Figure 1D) or the N-terminus of p35 (Figure 1 E). In addition, Figure 1 shows how a second cytokine may be fused to a single chain version of IL-12. Specifically, single chain IL-12 molecules may have p35 N-terminal to p40, with the second cytokine at the C-terminus (Figure 1 F) or the N-terminus (Figure 1 G). Alternatively, single chain IL-12 molecules may have p40 N-terminal to p35, with the second cytokine at the C-terminus (Figure 1 H) or the N-terminus (Figure 1I).

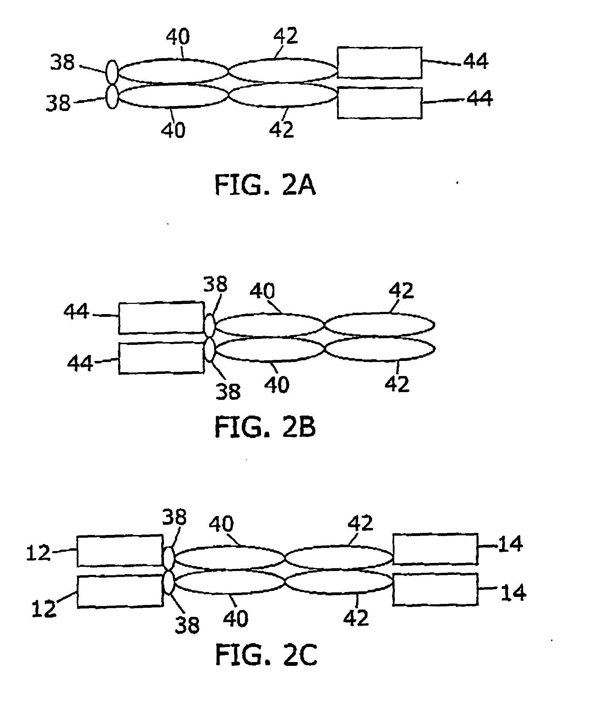

Figures 2A-2C schematically show how multiple-cytokine fusions (boxed) depicted in Figure 1 may be further fused to an Fc region of an antibody, shown here as a hinge (H), a CH2 domain and a CH3 domain (ovals). Specifically, any of the eight molecules of Figure 1 may be fused to either the C-terminus (Figure 2A) or N-terminus (Figure 2B) of the Fc region. In addition, the first cytokine and second cytokine (each boxed) need not be directly attached to each other, but can be connected through the Fc moiety (Figure 2C).

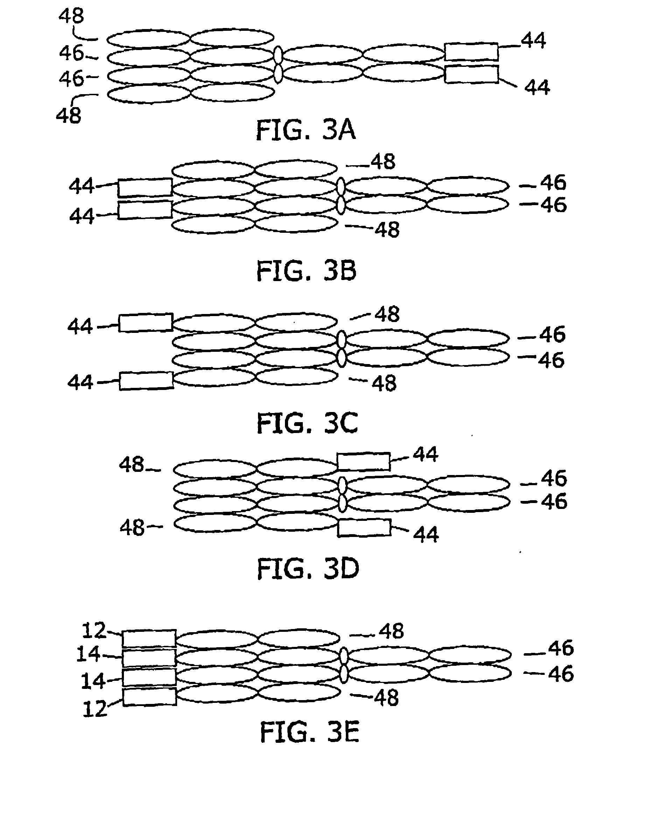

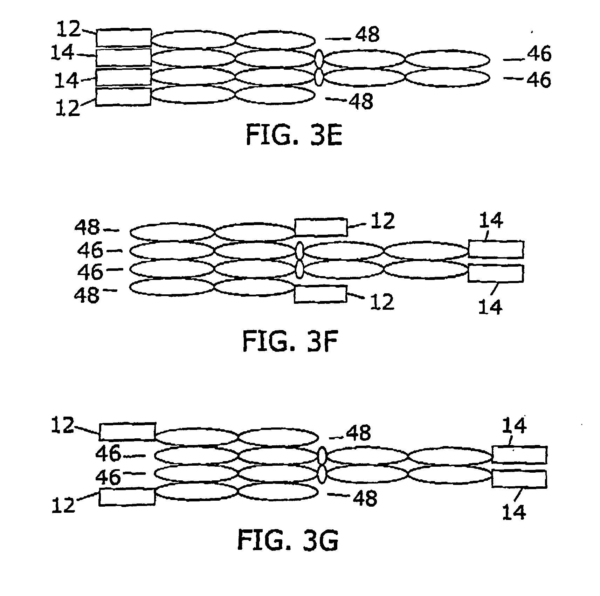

Figures 3A-3G schematically show a subset of the ways in which a multiple cytokine fusion protein may be further fused to an intact immunoglobulin such as an IgG. The heavy chain V region is shown as an oval labeled VH, the light chain V region is shown as an oval labeled VL, and constant regions are blank ovals. A multiple cytokine fusion, as illustrated in Figure 1, may be placed at the C-terminus of the heavy chain (Figure 2A), the N-terminus of the heavy chain (Figure 2B), the N-terminus of the light chain (Figure 2C), or the C-terminus of the light chain (Figure 2D). In addition, there are many ways in which a first and second cytokine could be separately attached at the N- and C-termini of the heavy and light chains; three of these are shown in Figures 3E-3G.

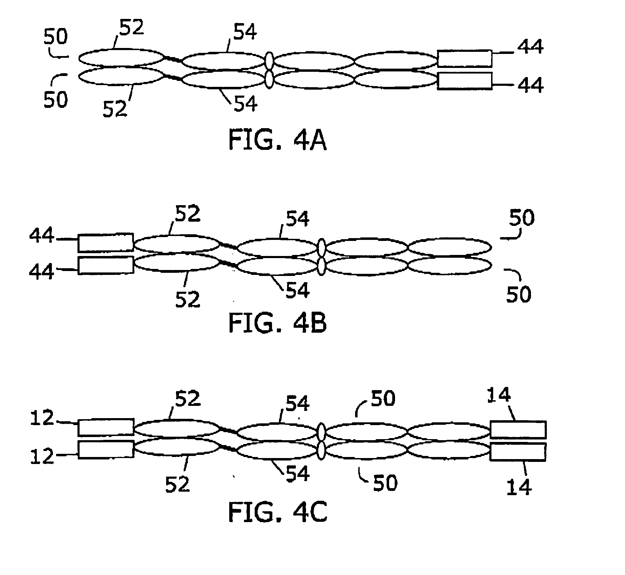

Figures 4A-4C schematically show how a first cytokine and a second cytokine may be fused to a "single-chain" antibody in which the variable light and variable heavy chains are fused, and the protein is expressed as a single polypeptide that then homodimerizes. Specifically, a multiple cytokine fusion may be placed at the C-terminus (Figure 4A), or the N-terminus (Figure 4B). In addition, the a first cytokine and second cytokine need not be directly attached, but can be connected through the single-chain antibody moiety (Figure 4C).

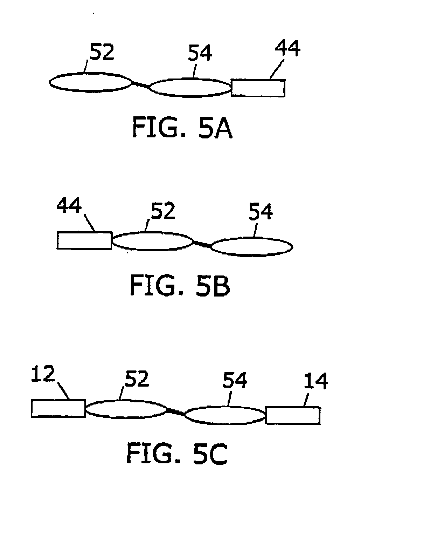

Figures 5A-5C schematically show how a first cytokine and a second cytokine may be fused to a single-chain Fv region consisting of the fused variable regions from a heavy chain and a light chain. Specifically, a first cytokine-cytokine fusion may be placed at the C-terminus (Figure 5A), or the N-terminus (Figure 5B). In addition, the first cytokine and second cytokine need not be directly attached, but can be connected through the single-chain Fv moiety (Figure 5C).

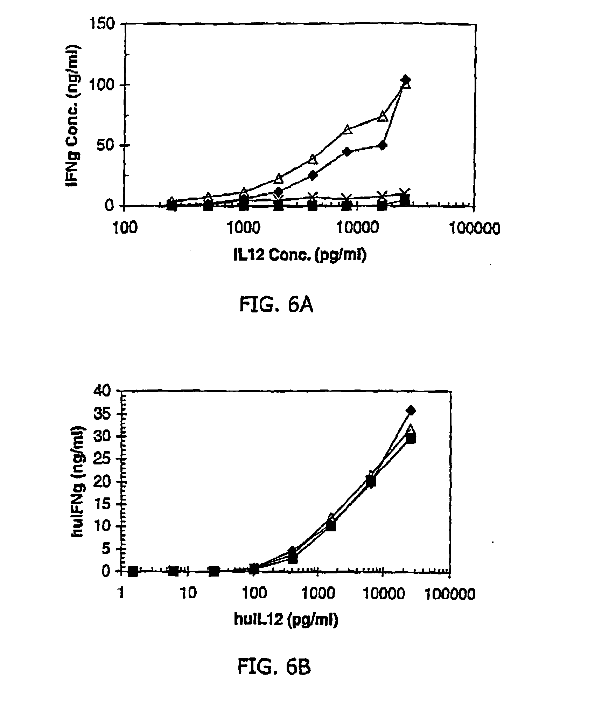

Figures 6A and 6B show the synergy between IL-12 and IL-2 in the induction of IFN- by human peripheral blood mononuclear cells (PBMCs) in response to the separate cytokines or fusion proteins. In Figure 6A, cells were treated with human IL-12 before (squares) or after phytohemagglutinin activation (X's), or with IL-12-IL-2 fusion protein before (diamonds) or after phytohemagglutinin activation (triangles). Figure 6B shows an experiment in which cells were treated with a mixture of IL-12 plus IL-2 added in a 1:1 molar ratio (black diamonds), human Fc-IL-12-IL-2 fusion protein (gray squares), and human antibody-IL-12-IL-2 fusion protein (light gray triangles). The X axis indicates the concentration of IL-12 in pg/ml, whether present as an intact protein or as a fusion protein. The y-axis indicates IFN- concentration (in ng/ml), which was assayed by ELISA.

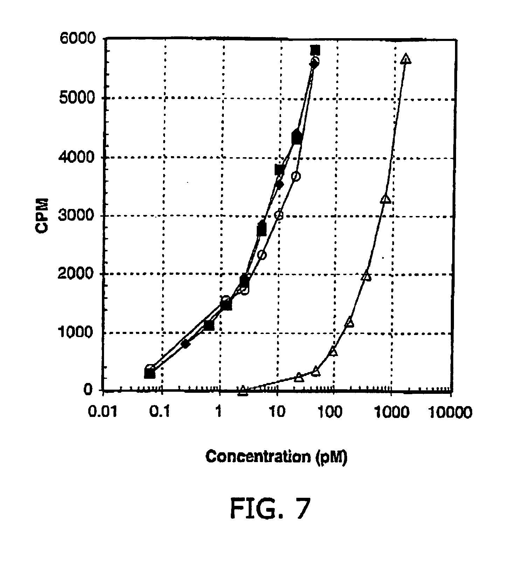

Figure 7 shows a typical IL-12 bioassay that separately measures activity of a fusion protein and compares it to that of a non-fused IL-12 molecule. What is depicted is the stimulation of ³H-thymidine uptake of human PBMCs in response to murine IL-12 (white circles), to a mixture of murine IL-12 plus IL-2 added in a 1:1 molar ratio (black squares), to murine IL-2 (white triangles), and to an antibody-murine IL-12-IL-2 fusion protein (black diamonds). The X axis indicates the concentration (pM) of monomeric cytokine(s), whether present as an intact protein or as a fusion protein; the y-axis indicates cpm of tritiated thymidine incorporation.

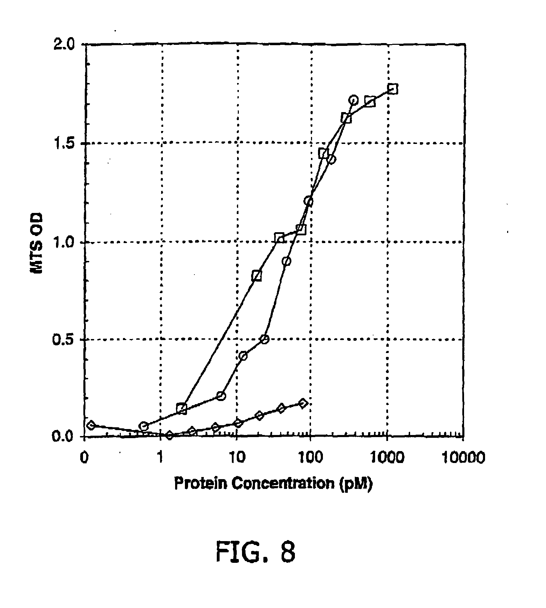

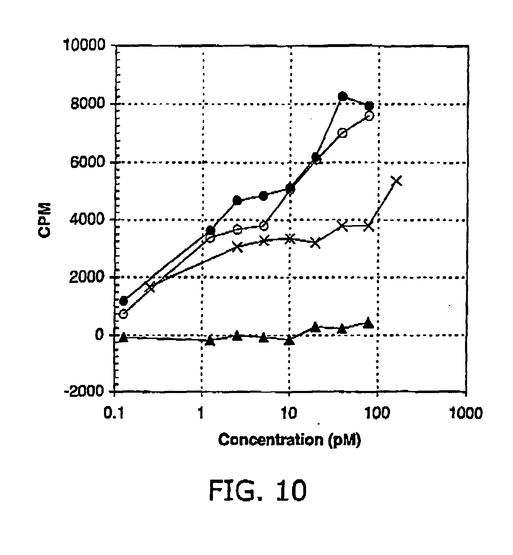

Figure 8 shows a standard IL-2 bioactivity assay. The graph shows the stimulation of mouse CTLL cell proliferation, in response to murine IL-2 (circles), to an antibody-murine IL-12-IL-2 fusion protein (diamonds), and murine IL-12 (squares). The x axis indicates the concentration (pM) of monomeric cytokine(s), whether present as an intact protein or as a fusion protein. Cells were incubated in medium containing various amounts of cytokine or fusion protein for 48 hours, then assayed for viable cell number using the MTT/MTS assay. The y-axis indicates the absorbance at 490 nanometers in units of optical density (OD).

Figure 9 shows the stimulation of ³H-thymidine uptake by human PBMCs in response to murine IL-12 (white circles), to a mixture of murine IL-12 plus IL-2 added in a 1:1 molar ratio (black circles), to a murine Fc-single-chain-IL-12-IL-2 fusion protein (black triangles), and to murine single-chain IL-12 fused to murine IL-2 (black diamonds). The x axis indicates the concentration (pM) of monomeric cytokine(s), whether present as an intact protein or as a fusion protein; the y-axis indicates cpm of tritiated thymidine incorporation.

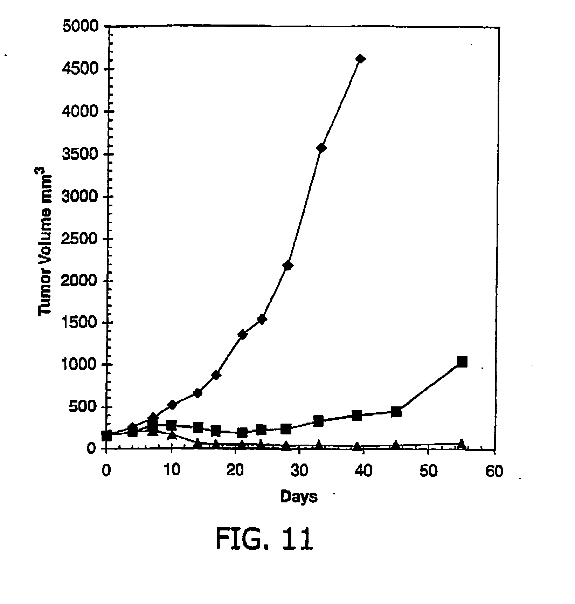

Figure 11 shows the effect of antibody-cytokine-cytokine fusion protein treatment of Balb/C mice bearing subcutaneous tumors derived from CT26 colon carcinoma cells that were engineered to express human EpCAM, the antigen for KS-1/4. Black diamonds indicate average tumor volumes in mice that were injected with PBS as controls on days 0, 1, 2, 3, and 4. Triangles indicate average tumor volumes in mice treated with 6 micrograms of KS-IL-12-IL-2. Squares indicate average tumor volumes in mice treated with 3.4 micrograms of KS-IL2 and 5.3 micrograms of KS-IL12. Intratumoral injections were performed. The x-axis indicates the number of days elapsed following the first injection; the y-axis indicates the average tumor volume in cubic milliliters.

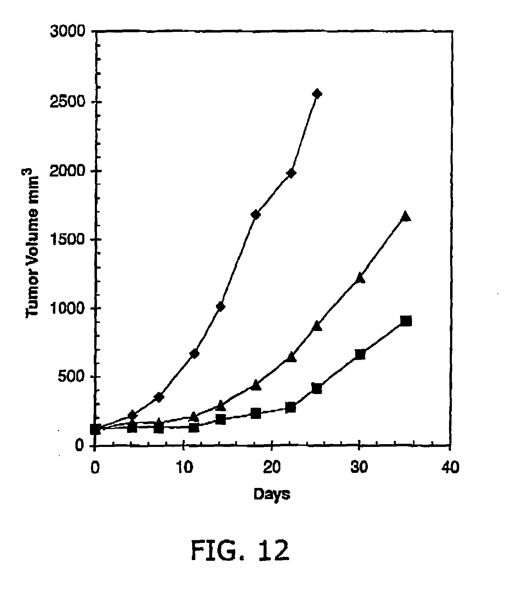

Figure 12 shows the effect of antibody-cytokine-cytokine fusion protein treatment of SCID mice bearing subcutaneous tumors derived from CT26 colon carcinoma cells that were engineered to express human EpCAM. Diamonds indicate average tumor volumes in mice that were injected with PBS as controls on days 0, 1, 2, 3, and 4. Triangles indicate average tumor volumes in mice treated with 6 micrograms of KS-IL-12-IL-2. Squares indicate average tumor volumes in mice treated with 3.4 micrograms of KS-IL2 and 5.3 micrograms of KS-IL12. Intratumoral injections were performed. The x-axis indicates the number of days elapsed following the first injection; the y-axis indicates the average tumor volume in cubic milliliters.

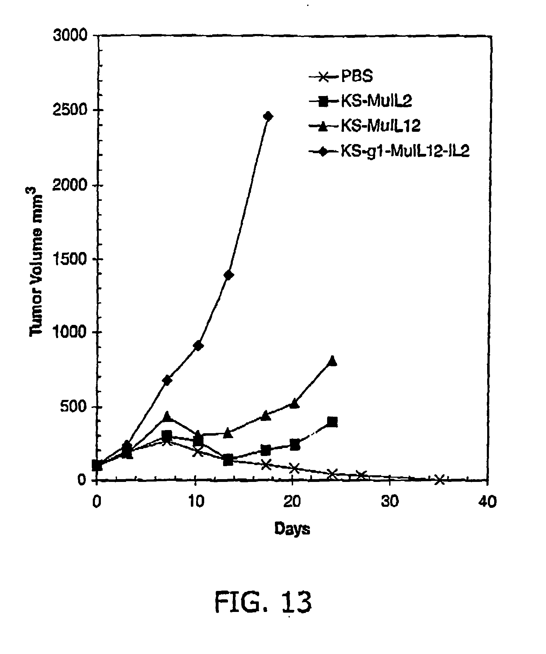

Figure 13 compares the effect of antibody-cytokine and antibody-cytokine-cytokine fusion protein treatment of mice bearing subcutaneous tumors of Lewis lung carcinoma (LLC) cells that were engineered to express human EpCAM. Diamonds indicate average tumor volumes in mice that were injected intratumorally with PBS as controls on days 0, 1, 2, 3, and 4. Squares indicate average tumor volumes in mice injected intratumorally with 20 micrograms of KS-IL2 on days 0, 1, 2, 3, and 4. Triangles indicate average tumor volumes in mice injected intratumorally with 20 micrograms of KS-IL12 on days 0, 1, 2, 3, and 4. X's indicate average tumor volumes in mice injected intratumorally with 20 micrograms of KS-IL-12-IL-2 on days 0, 1, 2, 3, and 4. The x-axis indicates the number of days elapsed following the first injection; the y-axis indicates the average tumor volume in cubic milliliters.

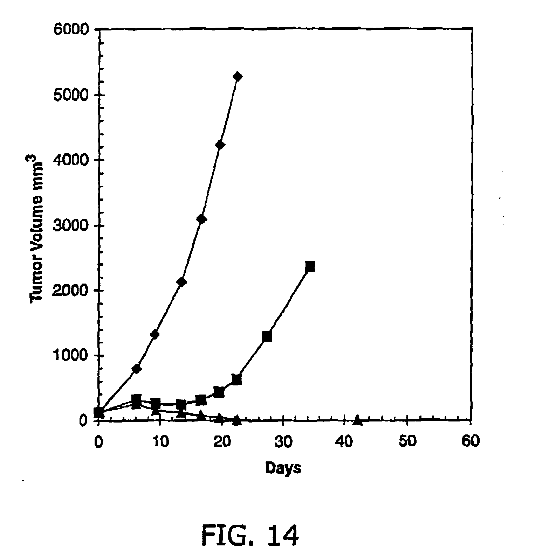

Figure 14 shows the effect of antibody-cytokine-cytokine fusion protein treatment of mice bearing subcutaneous tumors derived from Lewis lung carcinoma cells that were engineered to express human EpCAM. Diamonds indicate average tumor volumes in mice that were injected with PBS as controls on days 0, 1, 2, 3, and 4. Triangles indicate average tumor volumes in mice treated with 20 micrograms of KS-IL-12-IL-2. Squares indicate average tumor volumes in mice treated with 11.5 micrograms of KS-IL2 and 18 micrograms of KS-IL12. Intratumoral injections were performed. The x-axis indicates the number of days elapsed following the first injection; the y-axis indicates the average tumor volume in cubic milliliters.

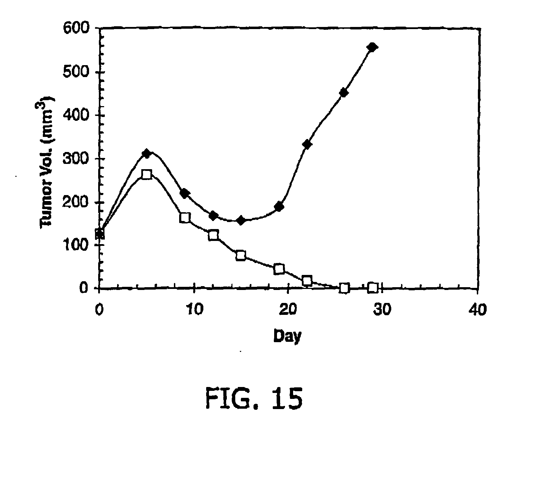

Figure 15 shows the effect of antibody-cytokine-cytokine fusion protein treatment of mice bearing subcutaneous tumors derived from Lewis lung carcinoma cells that either do or do not express human EpCAM. Black squares indicate average tumor volumes in mice bearing LLC/KSA-derived tumors. Black diamonds indicate average tumor volumes in mice bearing LLC-derived tumors. Mice were treated with 20 micrograms of KS-IL12-IL2 on days 0, 1, 2, 3 , and 4. Intratumoral injections were performed. The x-axis indicates the number of days elapsed following the first injection; the y-axis indicates the average tumor volume in cubic milliliters.

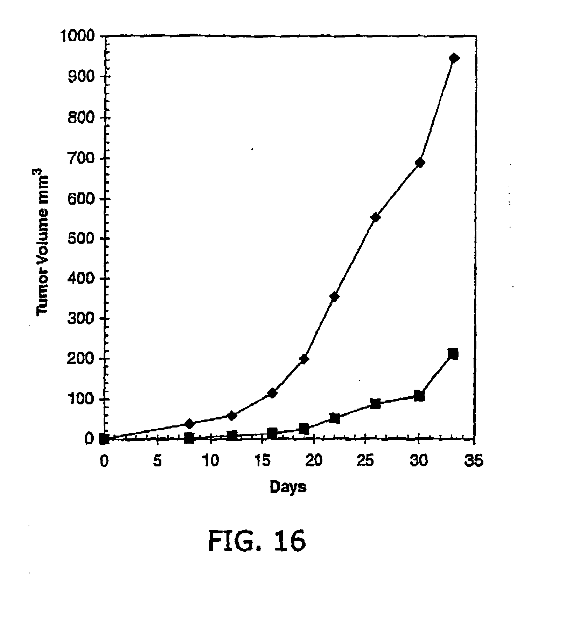

Figure 16 shows the effect of antibody-cytokine-cytokine fusion protein treatment of mice bearing subcutaneous tumors derived from Lewis lung carcinoma cells. About 10⁶ cells were injected subcutaneously on Day 0. Diamonds indicate average tumor volumes in naïve mice. Squares indicate average tumor volumes in mice that had previously had subcutaneous tumors derived from Lewis lung carcinoma cells that were engineered to express human EpCAM, and had been cured of these tumors by treatment with KS-IL12-IL2. The x-axis indicates the number of days elapsed following the injection; the y-axis indicates the average tumor volume in cubic milliliters.

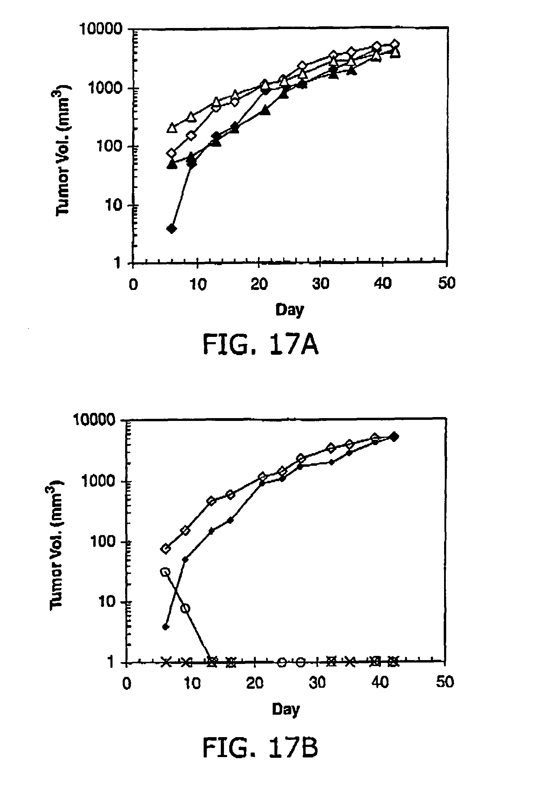

Figures 17A and 17B show the effect of single- or multiple-cytokine protein secretion by tumor cells on the ability of the cells to form tumors in an animal with a normal immune system. In Figure 17A, four sets of mice are compared: C57BL/6 mice injected s. c. with 1x10⁶ LLC tumor cells (black diamonds); C57BL/6 mice injected s. c. with 5x10⁶ LLC tumor cells (white diamonds); C57BL/6 mice injected s. c. with 1x10⁶ LLC tumor cells expressing scIL-12 (black triangles); and C57BU6 mice injected s. c. with 5x10⁶ LLC tumor cells expressing scIL-12 (white triangles). Figure 17B compares C57BL/6 mice injected s. c. with 1x10⁶ LLC tumor cells (black diamonds); C57BU6 mice injected s. c. with 5x10⁶ LLC tumor cells (white diamonds); C57BL/6 mice injected s. c. with 1x10⁶ LLC tumor cells expressing scIL-12-IL-2 (X's); and C57BL/6 mice injected s. c. with 5x10⁶ LLC tumor cells expressing scIL-12 (white circles). The x-axis indicates number of days after injection of the tumor cells. The y-axis indicates the tumor volume in cubic millimeters.

### Detailed Description of the Invention

The invention provides protein molecules in which two distinct cytokines are fused or complexed. The protein complexes or fusion proteins include additional protein moieties, including moieties capable of multimerization and targeting such as antibody Fc regions and antibody regions that include antigen combining sites. The invention also provides nucleic acids encoding multiple-cytokine fusion proteins. The invention also provides methods for the construction of nucleic acids encoding multiple-cytokine fusion proteins, methods for production of multiple-cytokine fusion proteins, and methods for use of multiple-cytokine fusion proteins in treatment of diseases and medical conditions.

As used herein, "cytokine" refers to a secreted protein or active fragment or mutant thereof that modulates the activity of cells of the immune system. Examples of cytokines include the interleukins, interferons, chemokines, tumor necrosis factors, colony-stimulating factors for immune cell precursors, and so on.

As used herein, "heterodimeric cytokine" refers to a cytokine consisting of two distinct protein subunits. At present, IL-12 is the only naturally occuring heterodimeric cytokine that is known. However, artificial heterodimeric cytokines can be constructed. For example, IL-6 and a soluble fragment of IL-6R can be combined to form a heterodimeric cytokine, as can CNTF and CNTF-R alpha [Trinchieri (1994) Blood 84:4008].

As used herein, "interleukin-12" (IL-12) refers to the two-subunit cytokine consisting of a p35 and p40 subunit, or an active single-chain fusion of p35 and p40, or a species variant, fragment, or derivative thereof.

As used herein, "interleukin-2" (IL-2) refers to any mammalian IL-2, such as human IL-2, mouse IL-2, or an active species or allelic variant, fragment or derivative thereof.

As used herein, "immunoglobulin Fc region" means the carboxyl-terminal portion of an immunoglobulin heavy chain constant region, or an analog or portion thereof. For example, an immunoglobulin Fc region of IgG may comprise at least a portion of a hinge region, a CH2 domain, and a CH3 domain. In a preferred embodiment the Fc region includes at least a portion of a hinge region and a CH3 domain. In another preferred embodiment, the Fc region includes at least a CH2 domain and more preferably also includes at least a portion of a hinge region..

As used herein, "peptide linker" means one or more peptides used to couple two proteins together (*e.g*. a protein and an Fc region). The peptide linker often is a series of amino acids such as. e.g., predominantly glycine and/or serine. Preferably, the peptide linker is a mixed series of predominantly glycine and serine residues and is about 10-15 amino acids in length.

As used herein, the term "multimeric" refers to the stable association of two or more protein subunits through covalent or non-covalent interaction, e.g. disulphide bonding.

As used herein, the term "dimeric" refers to a specific multimeric molecule where two protein subunits are associated stably through covalent or non-covalent interactions. A stable complex is a complex with a dissociation rate, or off-rate, of at least several minutes (such that the complex would be stable long enough during *in vivo* use to reach a target tissue and have a biological effect. The Fc fragment itself typically forms a dimer of the heavy chain fragments comprising a portion of the hinge region, CH2 domain and/or CH3 domain. However, many protein ligands are known to bind to their receptors as a dimer. If a cytokine X dimerizes naturally, the X moiety in an Fc-X molecule will dimerize to a much greater extent, since the dimerization process is concentration dependent. The physical proximity of the two X moieties connected by Fc would make the dimerization an intramolecular process, greatly shifting the equilibrium in favor of the dimer and enhancing its binding to the receptor.

As used herein, "vector" means any nucleic acid comprising a nucleotide sequence competent to be incorporated into a host cell and to be recombined with and integrated into the host cell genome, or to replicate autonomously as an episome. Such vectors include linear nucleic acids, plasmids, phagemids, cosmids, RNA vectors, viral vectors and the like. Nonlimiting examples of a viral vector include a retrovirus, an adenovirus and an adeno-associated virus.

As used herein, "gene expression" or "expression of a protein" is understood to mean the transcription of the DNA sequence, translation of the mRNA transcript, and either secretion of a protein product or production of the protein product in an isolatable form.

As used herein, an "immunocytokine" is a fusion protein comprising an antibody and a cytokine, as disclosed in U.S. Patent No. 5,650,150.

As used herein, a "leader sequence" is a protein sequence that is attached, usually at the N-terminus, to a second protein sequence, and that directs the second protein sequence to be secreted from a cell. The leader sequence is usually cleaved and removed from the second protein sequence, which becomes the mature protein. The term "leader sequence" is generally synonymous with "signal sequence".

As used herein, "EpCAM" refers to epithelial cell adhesion molecule (Cirulli et al. [1998] 140:1519-1534), and is synonymous with "KSA," meaning the antigen bound by the monoclonal antibody KS-1/4. EpCAM is a cell surface protein that is abundantly expressed on cancer cells derived from epithelial cells.

As used herein, "KS-1/4" refers to a particular monoclonal antibody that binds to EpCAM.

As used herein, "KS-IL2," "KS-IL12," and "KS-IL12-IL-2" (and the like) refer to antibody-cytokine fusion proteins consisting of KS-1/4 with interleukin-2, KS-1/4 with interleukin-12, and KS-1/4 with both interleukin-12 and interleukin-2, respectively. Analogously named fusion protein constructs are also used herein. Because it is possible to fuse cytokines at several positions on an antibody molecule, a description such as "KS-IL12-IL2" refers to the class of proteins comprising KS-1/4 with both interleukin-12 and interleukin-2 fused at any possible position, unless explicitly stated otherwise.

Several illustrative embodiments of protein constructs embodying the invention are illustrated in Figures 1-5. Parts of the molecules diagrammed in Figures 2 - 5 are labeled 1A-1I, referring to the fusion proteins shown in Figures 1A-1I and illustrating that any of the fusion proteins from Figure 1 can be further fused to other proteins as indicated. Cytokines are shown as rectangles, constant regions of antibodies are shown as ovals, and the heavy chain variable region and light chain variable region are shown as labeled ovals.

The present invention describes protein complexes containing two different cytokines and optionally including additional protein moieties. A homodimeric cytokine (*e.g*. interferon alpha, interferon beta, interferon gamma, IL-5, IL-8, or the like), although it contains multiple subunits, is nevertheless a single cytokine. Similarly, a heterodimeric cytokine such as IL-12, although it contains subunits that are different, is a single cytokine. Furthermore, a heterodimeric form of normally homodimeric cytokines, such as a MCP-1/MCP-2 heterodimer, or of two alleles of a normally homodimeric cytokine (*e.g*., Zhang, J. Biol. Chem. [1994] 269:15918-24) is a single cytokine. The complexes of the present invention contain two different cytokines, each of which (*e.g*. IL-2 and IL-12; IL-4 and GM-CSF; MCP-1 and eotaxin; etc.) is capable of modulating an activity of a cell of the immune system.

Figure 1A depicts a preferred embodiment of the invention: in fusion protein 10, the C-terminus of a first cytokine 12 is fused to the N-terminus of a second cytokine 14, optionally through a linker region (not shown). In some embodiments of the invention, the protein complex of the invention contains at least two cytokines with significantly different serum half-lives. For example, using a small and a large protein will often result in a fusion protein with a circulating half-life characteristic of the larger protein. Therefore, in situations where the combined effects of IL-12 and a second cytokine are desired, it would be advantageous to express the two cytokines as a fusion protein of the general formula: IL-12-X or X-IL-12, where X is a second cytokine. Two particular advantages are seen. First, the serum half-life of the more rapidly cleared cytokine is extended. Second, the serum half-lives of both cytokines become very similar to each other.

A two-chain cytokine such as IL-12 can be fused to another cytokine at the N- or C-terminus of either the chain of the two-chain cytokine. In one embodiment, a second cytokine is fused at either the N-terminus or C-terminus of either the p35 or p40 subunit of IL-12 (Figures 1B-1E). In fusion protein 16 of Figure 1B, the N-terminus of a first cytokine 12 is fused to the C-terminus of the IL-12 subunit p40 18. Subunit p40 18 is connected to IL-12 subunit p35 20 by a covalent bond 22. In fusion protein 24 of Figure 1C, the N-terminus of p40 subunit 18 is fused to the C-terminus of first cytokine 12 and is connected to p35 subunit 20 by a covalent bond 22. Figure 1D depicts fusion protein 26 in which the N-terminus of first cytokine 12 is fused to the C-terminus of p35 subunit 20, which is connected by covalent bond 22 to p40 subunit 18. In Figure 1E, fusion protein 28 includes p35 subunit 20, fused at its N-terminus to the C-terminus of first cytokine 12 and connected by covalent bond 22 to p40 subunit 18.

In a second embodiment, the subunits of IL-12 may be fused to form a single-chain protein, scIL-12, with either the p35 subunit or the p40 subunit at the N-terminal position; the second cytokine may be attached to the N- or C-terminus of the resulting scIL-12 (Figures 1F-1I). Thus, in a preferred embodiment depicted in Figure 1F, fusion protein 30 contains single-chain IL-12, in which the N-terminus of p40 subunit 18 is fused to the C-terminus of p35 subunit 20, optionally through a peptide linker. In this embodiment, the N-terminus of cytokine 12 is fused to the C-terminus of p40 subunit 18. In the embodiment shown in Figure 1G, the N-terminus of p35 subunit 20 is fused to the C-terminus of cytokine 12, optionally through a peptide linker. Figures 1H and 1I show fusion proteins 34 and 36 containing another single-chain version of IL-12 in which the N-terminus of the p35 subunit is fused to the C-terminus of the p40 subunit, optionally through a peptide linker. In fusion protein 34, shown in Figure 1H, the N-terminus of cytokine 12 is fused to the C-terminus of p35 subunit 20. In fusion protein 36, shown in Figure 1I, the N-terminus of p40 subunit 18 is fused to the C-terminus of cytokine 12. In a highly preferred embodiment, IL-12 is fused to IL-2.

The production of such molecules is further illustrated in the examples.

It is often convenient to express heteromultimeric molecules, such as IL-12 or an antibody, as single-chain molecules in which the non-identical subunits are connected by short amino acid linkers [Huston et al (1988) Proc. Nat. Acad. Sci. 85: 5879; Lieschke et al. (1997) Nat Biotechnol. 15:35; Lieschke; G. J. and Mulligan; R. C., U.S. Patent No. 5,891,680]. A gene fusion is constructed, and then the desired protein can be expressed in cells containing a single recombinant DNA construct. Such single-chain versions of a heteromultimeric cytokine can be further fused to a second cytokine, which still allows a fusion protein with the desired activities to be expressed from a single recombinant DNA construct. The expression of such molecules is illustrated in the examples.

The invention also describes fusion proteins in which multiple distinct, fused cytokines are further fused to a protein capable of forming multimers, such as homodimers or heterodimers. The advantage of such a molecule is that the potency of one or more of the cytokines may be enhanced by dimerization. In some cases, enhancement of potency by dimerization can occur because the cytokine binds to its receptor as a dimer. In one embodiment, multiple cytokines are fused to a portion of an antibody molecule, such as an Fc region (Figure 2). In another embodiment, IL-12 and a second cytokine are fused to the homodimerizing protein moiety. In a preferred embodiment, the second cytokine is IL-2 . The fusion proteins may be created in a variety of ways, reflecting all the various orderings of several distinct protein moieties from the N- to C-termini in a fusion protein. For example when interleukin-12 and a second cytokine are fused to an Fc region, the two cytokines may be both fused in any order to the N- or C-terminus of the Fc region, or one cytokine may be fused at the N-terminus and the other at the C-terminus.

Some of these permutations are illustrated in Figure 2. For example, in the embodiment shown in Figure 2A, a fusion protein 44 of the invention is fused to the C-terminus of an Fc region containing hinge region 38, CH2 region 40 and CH3 region 42. Fusion protein 44 could have a variety of structures, including, for example, the structures of fusion proteins 10, 16, 24, 26, 28, 30, 32, 34, or 36 depicted in Figures 1A-1I. If fusion protein 44 has more than one N-terminus and C-terminus, as in fusion proteins 16, 24, 26, and 28, the Fc region could be fused to either N-terminus of fusion protein 44. As shown in Figure 2B, fusion protein 44 could be fused to the N-terminus of an Fc region. In the embodiment shown in Figure 2C, a first cytokine 12 could be fused to the N-terminus of an Fc region, and a second cytokine 14 could be fused to the C-terminus of the Fc region.

### Targeting

The invention also describes fusion proteins in which two cytokines are attached to a protein that is capable of localizing the cytokines to a particular target molecule, cell, or bodily location. The preferred molecule with localizing capability is an antibody or a moiety comprising the antigen-binding variable regions of an antibody. However, other localizing molecules, or domains thereof, may be used, such as specific ligands or receptors, naturally occurring binding proteins, enzymes that bind to particular substrates, artificially generated peptides that have been selected for a particular binding or localizing capability, peptides with distinctive physico-chemical properties that result in a targeting capability, proteins that possess a targeting capability by virtue of binding to another molecule that is targeted, or other types of proteins. In the case of fusing two cytokines to a targeting molecule, a preferred first cytokine is IL-12. When IL-12 is used, a preferred second cytokine is IL-2.

In the case of an antibody, there are a large number of ways in which two or more cytokines can be fused, because there are several possible sites of attachment. For example, an IgG antibody consists of two heavy and two light chains. The two cytokines may be fused to each other and then fused to an N- or C-terminus of either the heavy or the light chain. Alternatively, each cytokine may be fused separately to one of the N- or C-termini on the antibody molecule.

Figure 3 illustrates a subset of ways in two cytokines may be fused to an antibody molecule. For example, referring to Figure 3A, a fusion protein 44 of the invention could be fused to the C-terminus of an immunoglobulin heavy chain 46, which is associated with an immunoglobulin light chain 48. As in Figure 2, fusion protein 44 can have a variety of structures, including, for example, the structures of fusion proteins 10, 16, 24, 26, 28, 30, 32, 34, or 36 depicted in Figures 1A-1I. As shown in Figure 3B, a fusion protein 44 could be fused to the N-terminus of an immunoglobulin heavy chain 46 associated with an immunoglobulin light chain 48. In the embodiments shown in Figures 3C and 3D, fusion protein 44 is fused to the N-terminus (Figure 3C) or the C-terminus (Figure 3D) of an immunoglobulin light chain 48 associated with an immunoglobulin heavy chain 46. As shown in Figures 3E and 3F, a first cytokine 12 may be fused to an immunoglobulin light chain 48 associated with an immunoglobulin heavy chain 46 fused to a second cytokine 14. The cytokines 12 and 14 may be fused to the N-termini (Figure 3E) or the C-termini (Figure 3F) of the immunoglobulin chains. Alternatively, as in Figure 3G, first cytokine 12 may be fused to the N-terminus of the immunoglobulin light chain 48 while the second cytokine 14 is fused to the C-terminus of the immunoglobulin heavy chain 46.

### Fusions to single-chain antibodies

It is sometimes convenient to express antibodies as single-chain molecules. The invention also provides fusion proteins in which two or more cytokines are fused to a single-chain antibody. This has the advantage of reducing the number of the DNA constructs used when expressing the desired fusion protein, which may be especially useful in gene therapy. In particular, if the cytokines are single-chain molecules, then fusion of the cytokines to the single-chain antibody will allow expression of the fusion protein as a single protein chain.

As shown in Figures 4A-4C, in some embodiments, cytokines can be fused to the single-chain antibody at its N-terminus, its C-terminus, or at both termini. For example, as shown in Figure 4A, fusion protein 44 can be fused to the C-terminus of single-chain antibody 50 having light chain variable region 52 and heavy chain variable region 54. As shown in Figure 4B, fusion protein 44 can also be fused to the N-terminus of single-chain antibody 50. In the embodiment shown in Figure 4C, a first cytokine 12 is fused to the N-terminus of single-chain antibody 50, and a second cytokine 14 is fused to the C-terminus of single-chain antibody 50.

A preferred embodiment comprises a fusion of IL-12 and a second cytokine to the single-chain antibody. A more preferred embodiment comprises IL-2 as the second cytokine.

The constant regions of antibodies have the potential to mediate a variety of effector functions. For example IgG1 mediates complement fixation, ADCC, and binding to Fc receptor. The position at which the cytokine is fused may alter the antibody constant region's effector function, which is useful if modulation of these effector functions is desired.

In some cases it may be desirable to construct a fusion of two or more cytokines to a moiety with the targeting region of an antibody, but without the constant regions. Such a fusion protein is smaller than a fusion of a complete antibody to two or more cytokines, which may be advantageous for certain purposes. In addition, such a fusion protein will lack one or more of the effector functions of an intact antibody, but will retain the targeting capability of an antibody.

The invention therefore features fusion proteins in which two or more cytokines are fused to a single-chain Fv region. As shown in the embodiments depicted in Figures 5A-5C, two cytokines may be fused to the N-terminus or the C-terminus of the Fv region, or one cytokine to each terminus. For example, as shown in Figure 5A, a fusion protein 44 of the invention may be fused to the C-terminus of a single-chain Fv region containing an immunoglobulin light chain variable region 52 and an immunoglobulin heavy chain variable region 54. A fusion protein 44 may also be fused to the N-terminus of an Fv region as shown in Figure 5B. As shown in Figure 5C, a first cytokine 12 may be fused to the N-terminus of an Fv region, and a second cytokine 14 may be fused to the C-terminus of the Fv region.

### Antibodies as heterodimeric vehicles for multiple cytokines

In some circumstances, it is desirable to construct a fusion of two or more cytokines in which, for two of the cytokines, the same end of the protein is essential for activity. For example, it may be that the naturally occurring N-terminus of two different cytokines is essential for the activity of each cytokine. It is not possible to construct a single polypeptide chain fusion protein in which both cytokine moieties would be active.

Antibodies are heterodimeric proteins consisting of heavy and light chains that are covalently linked by disulfide bonds. If it is desired to construct a multiple cytokine fusion protein with two cytokine moieties that both require an intact, unfused N-terminus, it is preferable to separately fuse the two cytokines to the N-termini of the heavy and light chains of an antibody (Figure 3E). Similarly, if it is desired to construct a multiple cytokine fusion protein with two cytokine moieties that both require an intact, unfused C-terminus, it is preferable to separately fuse the two cytokines to the C-termini of the heavy and light chains of an antibody (Figure 3F). If the antibody is used solely as a vehicle to connect two cytokines in this manner. it may be useful to mutate or delete those portions of the antibody that confer additional properties related to immune function. For example, it may be preferable to use an Fab region as a vehicle, since the Fab region retains the heterodimerization feature of an antibody but lacks the functions characteristic of the Fc region. It may also be useful to use an antibody or antibody fragment in which the antigen combining site is non-functional.

Fusions of multiple cytokines to antibodies combine many of the novel features of the invention. In antibody-multiple cytokine fusions, the serum half-life of the cytokines is equalized and extended; the activity of both cytokines is localized to a target and the especially toxic effects due to systemic administration of multiple, synergistically acting cytokines are avoided; each cytokine is effectively dimerized or multimerized; and the cytokines do not need to be directly fused but may be fused to different sites on the heavy and light chains of the antibody molecule.

In designing a fusion protein comprising multiple cytokines and an antibody, there are a number of options and configurations that can be distinguished by routine experimentation. Structural biology considerations are also useful. For example, many cytokines fall into a class termed 4-helix bundles. These structures consist of four alpha helices and have the N-terminus and C-terminus in the same vicinity. In general, the face of a cytokine around the N- and C-terminus is not used in binding to a cytokine receptor, so either terminus can be used for fusion to and antibody or to a second cytokine. However, it is sometimes difficult to directly fuse both the N- and C-terminus of a 4-helix bundle cytokine to different moieties, for steric reasons. When it is desirable to fuse two different 4-helix bundle cytokines to an antibody, it is therefore useful to fuse each cytokine to a different site on the antibody. Alternatively, if it is necessary to construct a polypeptide chain of the form Ig chain-cytokine-cytokine, one or more flexible linkers may be used to overcome the steric problems.

Instead of an antibody, it is also possible to use other secreted heterodimeric molecules to carry multiple cytokines. For example, a complex including prostate-specific antigen and the protease inhibitor with which it complexes, the IgA heavy chain and the J chain, members of the TGF-beta family and their astacin-like binding partners, or IL-12 could be used.

### Nucleic acids

The invention also features nucleic acids capable of expressing each of the above types of proteins. These include nucleic acids encoding fusion proteins comprising two or more cytokines, fusions comprising two or more cytokines and a dimerization domain such as an Fc region, fusions comprising two or more cytokines fused to an antibody, and two or more cytokines fused to an Fv region. Preferred forms of the nucleic acids are DNA vectors from which the fusion proteins can be expressed in either bacteria or mammalian cells. For fusion proteins that comprise multiple polypeptide chains, more than one encoding nucleic acid may be used. Alternatively, it may be useful to place two or more fusion protein coding sequences on a single nucleic acid molecule. The Examples illustrate particular forms of the featured nucleic acids encoding multiple cytokines.

The nucleic acids of the invention are particularly useful for expression of multiple cytokine fusion proteins, for either the production of these proteins or for gene therapy purposes.

Methods for synthesizing useful embodiments of the invention, as well as assays useful for testing their pharmacological activities, are described in the Examples.

The present invention also provides pharmaceutical compositions and methods of their use in treatment and prevention of a wide variety of diseases, including but not limited to treatment of various infections and cancer, and vaccination against various diseases.

Multiple cytokine fusion proteins can be used to treat bacterial, parasitic, fungal, or viral infections, or cancer. For example, IL-12 is known to have a protective effect in many types of infections, including but not limited to infections with the bacterium *Listeria monocytogenes;* the parasites *Toxoplasma gondii, Leishmania major,* and *Schistosoma mansoni;* the fungus *Candida albicans;* and the viruses choriomeningitis virus and cytomegalovirus. Since cytokines generally act in combination, it is often useful to use fusion proteins comprising two or more cytokines that are known to act synergistically. For example, since IL-2 potentiates the effects of IL-12, it is useful to combine these cytokines in treatment of bacterial, parasitic, fungal and viral diseases.

A preferred method of treatment of infectious disease is to use multiple cytokine fusion proteins that are further fused to a targeting agent that places the multiple cytokine at the site of infection. Various targeting strategies are described below.

The pharmaceutical compositions of the invention may be used in the form of solid, semisolid, or liquid dosage forms, such as, for example, pills, capsules, powders, liquids, suspensions, or the like, preferably in unit dosage forms suitable for administration of precise dosages. The compositions will include a conventional pharmaceutical carrier or excipient and, in addition, may include other medicinal agents, pharmaceuticals agents, carriers, adjuvants, etc. Such excipients may include other proteins, such as, for example, human serum albumin or plasma proteins. Actual methods of preparing such dosage forms are known or will be apparent to those skilled in the art. The composition or formulation to be administered will, in any event, contain a quantity of the active component(s) in an amount effective to achieve the desired effect in the subject being treated.

Administration of the compositions hereof can be via any of the accepted modes of administration for agents that exhibit such activity. These methods include oral, parenteral, or topical administration and otherwise systemic forms. Injection is a preferred method of administration.

The amount of active compound administered will, of course, be dependent on the subject being treated, the severity of the affliction, the manner of administration, and the judgment of the prescribing physician.

As described above, cytokines such as IL-2, IL-12, GM-CSF, IL-4, and others have been investigated for treatment of cancer. Under some circumstances it is advantageous to use a multiple cytokine fusion protein in treatment of cancer, for reasons of simpler administration, increased serum half-life of one of the component cytokines, and/or superior modulation of the relative activities of the two cytokines.

A preferred method of treatment of cancer is to target the cytokines to a particular organ or tissue, so the effect of the cytokines may be concentrated and the side effects of systemic distribution may be avoided. For example, fusions of multiple cytokines to an Fc region are expected to be concentrated to the liver, which may be useful in treatment of cancer limited to the liver. A more preferred method is to use a multiple cytokine fusion protein that is further fused to a targeting agent such as an antibody. In particular, the antibodies KS-1/4 and 14.18 are directed against tumor-specific antigens (Varki NM et al., Cancer Res [1984] 44:681-7; Gillies et al., Journal of Immunological Methods 125:191 [1989]; U.S. Patent Nos. 4,975,369 and 5,650,150). When using antibody-multiple cytokine fusion, it is often useful to investigate the type of tumor and choose an antibody directed against an antigen that is likely to be present on that type of tumor. For example, it may be useful to characterize the tumor by FACS analysis, Western blot, examination of the tumor's DNA, or simply identifying the type of the tumor cell. Such methods of tumor characterization are well known to those skilled in the art of tumor characterization, such as oncologists and tumor biologists. It is also possible to target multiple cytokine fusion proteins by a variety of other means, such as fusion to specific ligands or receptor moieties, fusion to peptide aptamers with pre-selected binding activities, chemical conjugation to small molecules with localizing characteristics, and so on. These targeting methods may also be used for treatment of other diseases, such as infections.

### Treatment of cancer and other cellular disorders by gene therapy

The nucleic acids of the invention may be used as gene therapy agents for treatment of cancer and other diseases in which it is desirable to target the immune system to a specific cell type. For example, cancer cells are withdrawn from a human or animal, one or more nucleic acids encoding a multiple cytokine fusion protein are transfected into the cancer cells, and the cancer cells are then reintroduced into the human or animal. Alternatively, the DNA may be introduced into the cancer cell *in situ.* The human or animal then mounts an immune response to the cancer cells, which may cure or lessen the severity of the cancer. A multiple cytokine gene fusion, coupled to appropriate regulatory elements to promote expression in mammalian cells, may be transfected into the cancer cells by any of a variety of techniques, include the calcium phosphate method, a 'gene gun', adenovirus vectors, cationic liposomes, retroviral vectors, or any other efficient transfection method. The nucleic acid may encode a multiple cytokine fusion protein that is further fused to other moieties.

Anti-cancer gene therapy with a nucleic acid expressing a fusion of more than one fused cytokine, may be combined with other cancer treatments, such as treatments that may augment the immune-stimulating properties of the fused cytokine protein. For example, the nucleic acid of the invention may also express other protein moieties that may aid in the development of an immune response to antigens expressed by the cancer cells, or may be co-transfected with other nucleic acids expressing such protein moieties. In particular, nucleic acids expressing the B7 costimulatory surface protein may be cotransfected into the cancer cells [Robinson et al., U.S. Patent No. 5,738,852]. Transfection of cancer cells with a nucleic acid expressing multiple cytokine fusion may also be accompanied by treatment with an antibody or immunocytokine that targets the cancer cells [Lode et al. (1998) Proc. Nat. Acad. Sci. 95:2475]. Transfection of cancer cells with a nucleic acid expressing multiple cytokine fusion may also be accompanied by treatment with an angiogenesis blocker [Lode et al. (1999) Proc. Nat. Acad. Sci. 9:1591].

Therapies using additional immune stimulators and/or angiogenesis blockers may also be combined with systemic treatment with multiple cytokine fusion proteins. An advantage of co-treatment with additional immune stimulators or angiogenesis blockers is that these treatments, unlike DNA-damaging agents and cell-cycle blockers, do not kill immune cells that may be dividing due to stimulation by the multiple cytokine fusion protein.

A preferred embodiment of this gene therapy method is to introduce one or more nucleic acids encoding IL-12 and a second cytokine into cancer cells, and then reintroduce the cancer cells into the human or animal. The second cytokine is preferably IL-2 or GM-CSF.

The present invention provides novel vaccine compositions and methods of adjuvantation of vaccines intended to provide a protective cell-mediated immune response in vaccinated host mammals against certain pathogens, using as an adjuvant two or more cytokines that have been fused. For example, if a Th1 immune response is desired, multiple Th1-promoting cytokines may be fused and the resulting fusion protein administered to an animal in combination with an antigen.

In particular, IL-12 and IL-2 may be fused and administered with an antigen. Alternatively, IL-12 and IL-2 may be further fused to an antigenic protein itself and used to stimulate an immune response. In this case, the invention is directed to vaccines that rely on the host's cell-mediated immunity, i. e. the elicitation of cytotoxic T lymphocytes and activated phagocytes to provide protection against infection by a particular pathogen. It is especially useful to perform vaccinations with fusion proteins comprising IL-12, IL-2, and the antigen, as this combination directs a Th1 response against the antigen. Conventional adjuvants used in humans, such as alum, tend to induce a Th2 response.

### Examples

### Example 1: Construction of gene fusions capable of expressing cytokine-cytokine fusion proteins

To create multifunctional proteins having a plurality of cytokines, gene fusions between IL-12's p40 and IL-2, and between IL-12's p40 and GM-CSF were synthesized. In addition, the coding sequence for mature murine p35 (SEQ ID NO:1) was fused to a promoter and leader sequence that allow high levels of expression and efficient secretion. Coding sequences of murine p40-IL-2 and p40-GM-CSF are shown in SEQ ID NO:2 and SEQ ID NO:3, respectively. A human p40-IL-2 fusion was also constructed (SEQ ID NO:4). Fusions of a mouse Fc region of IgG2a to mouse p35 (SEQ ID NO:5) and of human p35 to a human Fc region of IgG1 (SEQ ID NO:6) were constructed, using expression plasmids previously disclosed (Lo et al. Protein Engineering 11:495-500 [1998]; Lo et al., U.S. Patent No. 5,726,087).

Fusion of mature mouse and human p35 to the C-terminus of the KS-1/4 antibody heavy chain is described by Gillies et al. (J. Immunology [1998] 160:6195-6203). Fusions of mature mouse and human p35 to the C-terminus of the 14.18 antibody heavy chain were constructed in an analogous manner (PCT International Publication WO99/29732).

The type of strategy discussed here to fuse p40 to IL-2 and to GM-CSF is generally applicable to the fusion of two or more cytokines. Specifically, the coding sequence of the most N-terminal moiety comprises a signal sequence for secretion, while the C-terminal moieties does not require a signal sequence. In some circumstances, it may be useful to place a coding sequence for a short peptide linker, preferably 10-15 amino acids long and rich in glycine and serine, between the coding sequences for the two cytokines. The DNA manipulations involved in generating all such types of fusions are within the level of skill in the art.

For example, details of the construction of a fusion between the murine IL-12 p40 subunit and murine IL-2 were as follows. Full length cDNA of the p40 subunit of murine IL-12 was cloned by PCR from mouse spleen cells activated with Concavalin A (5 □g/ml in culture medium for 3 days). The forward primer had the sequence AA GCT AGC ACC ATG TGT CCT CAG AAG CTA ACC (SEQ ID NO:7), in which a NheI site *GCTAGC* (residues 3-8 of SEQ ID NO:7) was placed upstream of the translation initiation codon ATG, and the reverse primer had the sequence *CTC* GAG CTA GGA TCG GAC CCT GCA GGG (SEQ ID NO:8), in which an XhoI site CTCGAG (residues 1-6 of SEQ ID NO:8) was placed immediately downstream of the translation stop codon TAG (anticodon CTA). After sequence verification, the NheI-XhoI fragment containing the mu-p40 cDNA with its native leader was ligated to the XbaI-XhoI digested expression vector pdCs [Lo et al. (1998) Protein Engineering 11:495-500]. The restriction sites NheI and XbaI have compatible sticky ends, and NheI site was used for the cloning of mu-p40 because mu-p40 has an internal XbaI site.

For the construction of DNA encoding mu-p40-muIL-2, an oligonucleotide linker was used to join the mu-p40 DNA via its PstI site (C TGC AG) to a SmaI-XhoI fragment containing the cDNA of mature murine IL-2. The DNA sequence at the junction of the fusion protein was C TGC AGG GTC CGA TCC CCG GGT AAA GCA CCC (SEQ ID NO:9), where C TGC AG (residues 1-6 of SEQ ID NO:9) is the PstI site, C CCG GG (residues 15-20 of SEQ ID NO:9) is the Smal site, TCC is the C-terminal amino acid residue of murine p40, and GCA is the N-terminal residue of mature murine IL-2.

The DNA encoding single-chain muIL12-muGMCSF was derived from the DNA construct encoding single-chain muIL12-muIL2 above by replacing the muIL2 cDNA by the muGMCSF cDNA at the SmaI site. The DNA sequence at the junction of single-chain muIL12 and muGMCSF was C TGC AGG GTC CGA TCC CCG GGA AAA GCA (SEQ ID NO:10), where C *TGC* AG (residues 1-6 of SEQ ID NO:10) is the PstI site, C CCG GG (residues 17-22 of SEQ ID NO:10) is the SmaI site, TCC is the C-terminal amino acid residue of murine p40, and GCA is the N-terminal residue of mature murine GMCSF.

### Example 2: Expression of IL-12 fusion proteins

1L-12-IL-2 fusion proteins were expressed as follows. Different combinations of the individual vectors encoding p40 fusions and vectors encoding proteins comprising p35 were co-transfected into human 293 epidermal carcinoma cells for transient expression of fusion proteins. DNA was purified using preparative kits (Wizard, Promega Inc.), ethanol-precipitated for sterilization and resuspended in sterile water.

For expression of biologically active IL-12 fusion protein heterodimers, different combinations of the individual vectors encoding fusion and non-fusion forms of the subunits were transiently expressed by co-transfection of human 293 epidermal carcinoma cells. DNA was purified using preparative kits (Wizard, Promega Inc.), ethanol precipitated for sterilization and resuspension in sterile water. Calcium phosphate precipitates were prepared by standard methods using 10 µg of DNA per ml (5 µg of each when two plasmids were co-transfected) and 0.5 ml/plate were added to cultures of 293 growing in 60 mm plates at approximately 70% confluency (MOLECULAR CLONING: A LABORATORY MANUAL, 2nd Ed., Sambrook, Fritsch and Maniatis, eds., Cold Spring Harbor Laboratory Press, 1989). After 16 hr, the medium containing the precipitate was removed and replaced with fresh medium. After 3 days, the supernatant was removed and analyzed for production of transfected gene expression by ELISA, biological determination of IL-12 activity, or immunoprecipitation and analysis on SDS gels of radioactively labeled proteins. For labeling, medium without methionine was used to replace the growth medium on the second day of culture and ³⁵S-methionine (100 µCi/ml) was added. After an additional 16 hr incubation, the media was harvested, clarified by centrifugation (5 min at 13,000 rpm in a table top microcentrifuge) and incubated with protein A Sepharose beads (10 µl of bead volume per ml of culture supernatant). After 1 hr at room temperature, the beads were washed by repeated centrifugation and resuspension in PBS buffer containing 1% Nonidet-P40 (NP-40). The final pellet was resuspended in SDS-containing gel buffer and boiled for 2 min. After removing the beads by centrifugation, the supernatant was divided into two aliquots. Reducing agent (5% 2-mercaptoethanol) was added to one sample and both samples were boiled for 5 min prior to loading on an SDS polyacrylamide gel. After electrophoresis the gel was exposed to X-ray film (autoradiography).

Transfections using the following expression plasmids were performed: mu.p35 plus mu.p40-IL-2, KS-1/4-mu.p35 plus mu.p40, KS-1/4-mu.p35 plus mu.p40.IL-2, 14.18.mu.p35 plus mu.p40-lL-2, hu.Fc-.p35 plus hu.p40-IL-2, KS-1/4-hu.p35 plus hu.p40-lL-2, and 14.18-hu.p35 plus hu.p40-lL-2, where "mu" refers to murine proteins and "hu" refers to human proteins.

When cells were metabolically labeled with ³⁵S-methionine and secreted proteins examined by reducing SDS gel electrophoresis and autoradiography, high level expression was observed in each case. Molecular weights of reduced fusion proteins were predicted based on the molecular weights of component proteins, as follows: p35 of IL-12, 35 kD; p40 of IL-12, 40 kD; IL-2, 16 kD; Fc, 32 kD; Ig heavy chain, 55 kD; and Ig light chain, 28 kD. Proteins migrating with approximately the predicted molecular weights were observed.

Stably transfected cell lines expressing multiple cytokine fusion proteins were also isolated. In the case of heterodimeric constructs, IL-12 p40-lL-2 or IL-12 p40-GM-CSF fusion protein encoding expression vectors as described earlier for the IL-12 p40 subunit alone (Gillies et al. [1998] J. Immunol. 160: 6195-62030). Transfected cell lines expressing the p40 fusion proteins were transfected a second time with expression vectors encoding either the 1L-12 p35 subunit, an Fc-p35 fusion protein or an antibody-p35 fusion protein expression vector as described (Gillies et al. [1998] J. Immunol. 160: 6195-62030).

Supernatant from stably transfected cells expressing human Fc-IL-12-IL-2 (*i.e.* expressing KS-p35 and p40-IL-2) was collected and the products were purified by binding to and elution from protein A Sepharose according to the manufacturer's procedures (Repligen, Needham, MA). The pure proteins were assayed by ELISA for IL-12 and IL-2 content. The results showed that the individual cytokine contents were approximately 4-fold different by mass which correlates to the 4-fold difference in molecular weight between IL-12 and IL-2. Similarly, assay of the products of transfected cells expressing human KS- IL-12-IL-2 by ELISA for IL-12 and IL-2 levels gave similar values of IL-12 and IL-2. Thus, within the accuracy of the ELISAs, the measured values indicate that IL-12 and IL-2 are being produced in about a 1:1 molar ratio. The same results were obtained with the IL-12-GM-CSF fusion proteins made either with the Fc or whole antibody.

### Example 3: Synergistic activity of fusion proteins in an IFN-γ induction assay

Biological activity of IL-12-IL-2 fusion proteins was measured in an IFN-□ induction assay using either resting or mitogen-activated human peripheral blood mononuclear cells (PBMCs) from human volunteers (Figure 6). IFN-γ production was measured by ELISA.

Human peripheral blood mononuclear cells (PBMCs) were obtained from healthy volunteers and were purified by centrifugation on a Ficoll-Hypaque (Pharmacia) gradient (1700 rpm for 20 min). The "buffy" coat containing the PBMC was diluted with serum-free culture medium (SF-RPMI) to a volume of 50 ml and collected by centrifugation at 1500 rpm for 5 min. After gradient centrifugation, cells were resuspended in cell culture medium containing 10% fetal bovine serum (RPMI-10) with or without phytohemagglutinin (PHA; 10 µg/ml) at a density of 5 x 10⁶ cells/ml and were cultured for 3 days at 37°C in a humidified CO₂ incubator. The cells were collected by centrifugation, washed three times with an equal volume of SF-RPMI and resuspended in fresh RPMI-10 (1 x 10⁶ cells /ml). Aliquots (100 µl) were dispensed into the wells of multiple 96-well plates to give a final cell number of 10⁵ per well. Test samples from culture medium were serially diluted in fresh culture medium and added to wells of the 96-well plate. Control wells received IL-12 (Figure 6A) or an equimolar mixture of commercial IL-2 and IL-12 (Figure 6B; cytokines purchased from R & D Systems). The plates were incubated for 48 hr at 37°C in a CO₂ incubator at which time aliquots (20 µl) were removed for analysis of IFN-γ concentration by ELISA, following the instructions of the manufacturer (Endogen, Inc., Woburn, MA USA).

In Figure 6A, the activities of the human IL-12-IL-2 fusion protein were compared with IL-12 alone. The results illustrate that IL-12 alone induced IFN-γ to moderate levels, while the IL-12-IL-2 fusion protein strongly induced IFN-γ synthesis. Since IL-2 is also known to be insufficient for IFN-□ synthesis, these results indicate that the IL-12 and IL-2 moieties are both functional within the fusion protein and function synergistically.

Next, the activities of an Fc-IL-12-IL-2 fusion protein, a KS-IL-12-IL-2 fusion protein, and a mixture consisting of 1:1 molar ratio of IL-12 to IL-2 were compared for their ability to induce IFN-γ. The results in Figure 6B indicate that the Fc-IL-12-IL-2 fusion protein and KS-IL-12-IL-2 fusion protein have about the same activity as an equimolar mixture of IL-12 and IL-2. The same results were obtained when the mouse forms of IL-2 and IL-12, constructed in the manner just described in Example 1 for the human forms, were used for the construction of fusion proteins.

### Example 4 IL-2 and IL-12 bioactivity of IL-12-IL-2 fusion proteins.

The activities of IL-2 and IL-12 in the fusion proteins were compared to the free cytokines in proliferation-based assays. The activity of a murine antibody 14.18-IL-12-IL-2 molecule was tested in a typical IL-12 proliferation assay. Human PBMCs were obtained from volunteers and cultured with 5 micrograms/ml of phytohemagglutinin-P for three days, washed with Hank's HBSS, and plated into microtiter plates at 10⁵ cells per well, according to a standard procedure (Gately, M. K., Chizzonite, R., and Presky, D. H. Current Protocols in Immunology [1995] pp. 6.16.1 - 6.16.15). Cells were incubated in the presence of various test proteins for 48 hours, and 0.3 microCuries of ³H-thymidine were added ten hours before determining levels of radioactive incorporation. IL-12 and an equimolar mixture of IL-12 and IL-2 stimulated incorporation of ³H-thymidine into cells in a dose-dependent manner, and the 14.18-IL-12-IL-2 fusion protein was about equally effective in stimulating incorporation of ³H-thymidine. IL-2 stimulated incorporation of ³H-thymidine only at higher molar concentrations, indicating that the observed incorporation of ³H-thymidine stimulated by the 14.18-IL-12-IL-2 fusion protein is due primarily to its IL-12 activity. Results are shown in Figure 7.

In addition, the biological activity of the IL-2 moiety was tested in a different cell proliferation assay, following a standard procedure (Davis, L. S., Lipsky, P. E., and Bottomly, K. Current Protocols in Molecular Immunology [1995] p. 6.3.1 - 6.3.7). The mouse CTLL-2 cell line depends on IL-2 for proliferation. The CTLL-2 cell line can also proliferate in response to IL-4, but is not responsive to IL-12. CTLL-2 cells in active log-phase growth were washed twice in medium lacking IL-2 and plated at about 1x10⁴ cells per well in microtiter wells in the presence of various amounts of commercial murine IL-2, murine 14.18-IL-12-IL-2 fusions protein, or commercial murine IL-12, and grown for 48 hours. At the end of the growth period, the number of viable cells was quantitated using the MTT/MTS assay. Figure 8 shows an experiment in which levels of IL-2, IL-12, or 14.18-IL-12-IL-2 fusion protein were varied. The results indicate that murine IL-2 and murine 14.18-IL-12-IL-2 fusion protein are about equally potent in stimulating proliferation, while increasing amounts of murine IL-12 caused no detectable stimulation of cell proliferation. This result indicates that the stimulation of CTLL-2 cell proliferation by 14.18-IL-12-IL-2 fusion protein is due to the IL-2 moiety and not the IL-12 moiety.

### Example 5: Constrution and expression of single-chain and multiple chain IL-12-/L-2 fusion proteins with and without antibody moieties

A single-chain murine IL-12-IL-2 fusion protein was constructed as follows. A p40-IL-2 coding sequence fusion was constructed by methods analogous to those used in construction of the human p40-IL-2 fusion in example 1. To connect the DNAs encoding the p35 and p40 subunits of IL-12 and generate a single coding sequence, a DNA encoding a linker was synthesized with an XhoI site at the 5' end and a BamHI site at the 3' end. The 5' end of the mature p40-IL-2 coding sequence was modified to introduce a restriction site, and then ligated to the 3' end of the linker. The 3' end of the murine p35 coding sequence was modified to generate a restriction site and ligated to the XhoI site of the linker. The cDNAs encoding single-chain mulL12 and mu-p40-mulL2, described in Example 1, were combined by using a convenient restriction site in p40, to give a third DNA construct encoding single-chain mulL12-mulL2. These steps were carried out using various vectors and DNA fragment isolations as needed. The sequence of the resulting murine p35-linker-p40-IL-2 coding region is SEQ ID NO:11.

At the same time, a corresponding single-chain murine IL-12 coding sequence was constructed by corresponding methods. The coding sequence is SEQ ID NO:12.

In addition, we further constructed a DNA sequence that encodes a murine IgG2a Fc region fused to the N-terminus of p3S-Unker-p40-IL-2. The coding sequence is SEQ ID NO:13.

Cultured 293 cells were transfected with expression plasmids encoding the murine single-chain Fo-IL-12-IL-2 and Fc-IL-12 proteins. Expression of fusion proteins was assayed as described in Example 2. Fc fusion proteins were purified by their binding to protein A Sepharose, and high levels of expression of Fc-IL-12-IL-2 and Fo-IL-12 were observed. Proteins were synthesized intact, as inferred by the apparent molecular weights from migration on SDS gels: Fc-IL-12-IL-2, 123 kD; and Fc-IL-12, 107 kD.

The KS-scIL 12-IL2 fusion protein described in Example 1 is a tetramer with two different polypeptide chains: the KS-1/4 light chain and the KS-1/4 heavy chain with the sclL 12-IL2 moiety at the C-terminus. To investigate which sites on an antibody molecule are suitable for attachment of cytokine moieties, a second fusion protein was constructed in which the KS-1/4 antibody, IL-12, and IL-2 moieties were in a configuration distinct from the KS-IL12-IL2 configuration in Example 1. This second protein was tetrameric and consisted of two different polypeptides. One polypeptide consisted of the light chain of the KS-1/4 antibody. The other polypeptide consists of a single-chain mulL12 fused to the mature N-terminus of the heavy chain of the KS1/4 antibody, followed by murine IL-2 at the carboxyl terminus of the heavy chain.

The cDNA encoding the p35 subunit of murine IL-12 was cloned by PCR from mouse spleen cells activated with Concanavalin A (5 □g/ml in culture medium for 3 days). The forward primer has the sequence *AAGCTT*GCTAGCAGC **ATG TGT CAA** TCA CGC TAC (SEQ ID NO:14), where a HindIII site *AAGCTT* (residues 1-6 of SEQ ID NO:14) is placed upstream of the translation initiation codon **ATG**, and the reverse primer has the sequence *CTCGAG* **CTT TCA** GGC GGA GCT CAG ATA GCC (SEQ ID NO:15), where an XhoI site *CTCGAG* (residues 1-6 of SEQ ID NO:15) is placed downstream of the translation stop codon TGA (anticodon **TCA**).

The DNA encoding the single-chain IL-12 comprises of the mup35 DNA joined to oligonucleotides encoding a linker rich in glycine and serine residues, followed by mup40 DNA. The resultant construct has the following sequence at the oligonucleotide junction: GCC ATG (SEQ ID NO:16)
where *G AGC TC* (residues 1-6 of SEQ ID NO:16) is a Sacl restriction site just upstream of the murine p35 translation stop codon, GCG encodes the C-terminal amino acid residue of murine p35, *GGA TCC* (residues 50-55 of SEQ ID NO:16) is a BamHI restriction site introduced to facilitate ligation, and **ATG** encodes the N-terminal residue of mature mu-p40.

The DNA encoding single-chain muIL12-KS Heavy chain-muGMCSF has the following sequence at the junction of mup40 and the mature N-terminus of KS heavy chain: GGG GGC *TCC TTA AGC CAG* (SEQ ID NO:18)
where *C TGC AG* (residues 1-6 of SEQ ID NO:18) is a Pstl site just upstream of the murine p40 translation stop codon, **TCC** encodes the C-terminal amino acid residue of murine p40, and **CAG** encodes the N-terminal residue of mature KS heavy chain. The resultant DNA encoding single-chain muIL12-KS Heavy chain-muIL2 was then coexpressed with the KS light chain.

To further investigate which ends of an antibody molecule are available for the generation of fusion junctions and to investigate how many distinct polypeptides can be assembled into a multiple cytokine fusion protein, a third protein containing KS-1/4, IL-12, and IL-2, namely IL12-KS(Light chain) +KS(heavy chain)-IL2, was expressed and tested for activity. This fusion protein is hexameric and comprises three different polypeptides. One polypeptide consists of the murine p35 fused to the light chain of the KS1/4 antibody. A second polypeptide consists of the heavy chain of the KS1/4 antibody fused to human IL-2 [Gillies et al. (1992) Proc. Natl. Acad. Sci. 89:1428], and a third polypeptide is the murine p40. Upon expression, two light chains and two heavy chains are disulphide bonded to form the tetrameric antibody-cytokine structure. In addition, the p35 at the N-terminus of the light chain also is disulphide bonded with the p40.

The DNA encoding the mup35-KS light chain has the following sequence at the junction: TTA AGC **GAG** (SEQ ID NO:20)
where *G AGC TC* (residues 1-6 of SEQ ID NO:20) is a SacI restriction site just upstream of the murine p35 translation stop codon, GCG encodes the C-terminal amino acid residue of murine p35, *GGA TCC* (residues 50-55 of SEQ ID NO:20)is a BamHI restriction site introduced to facilitate ligation, and **GAG** encodes the N-terminal amino acid residue of the light chain.

For expression of this hexameric fusion protein, a murine p40-expressing cell line was generated by transfection with an expression vector containing a neomycin resistance gene and selection by G418. The murine p40-expressing cell line was then transfected with an expression vector containing both the light chain and heavy chain transcription units and a dihydrofolate reductase selection marker, which allowed selection by methotrexate [Gillies et al. (1998) J. Immunol. 160:6195].

### Example 6: Activity of murine single-chain IL-12-IL-2 fusion proteins

The same methods used in Example 4 were used to test the activity of murine single-chain IL-12-IL-2 produced by transient expression. The amount of each cytokine in the cell culture supernatant was first determined by ELISA and used to set up a dose-response curve. The activities closely corresponded to what was found with the Fc and antibody IL-12-IL-2 fusion proteins and described above.

Specifically, the IL-12 activity of a murine single-chain (sc) IL-12-IL-2 and murine Fc-scIL-12-IL-2 molecules were tested in the human PBMC cell proliferation assay described in Example 4. IL-12 and an equimolar mixture of IL-12 and IL-2 stimulated incorporation of ³H-thymidine into cells in a dose-dependent manner. On a per mole basis, both scIL-12-IL-2 and Fc-scIL-12-IL-2 fusion proteins were about as effective as IL-12 in stimulating incorporation of ³H-thymidine (Figure 9). As described in Example 4, IL-2 stimulates incorporation of ³H-thymidine only at a much higher molar concentrations, indicating that the observed incorporation of ³H-thymidine stimulated by the scIL-12-IL-2 fusion proteins is due primarily to their IL-12 activity.

In addition, the biological activity of the IL-2 moiety in the scIL-12-IL-2 fusion proteins was tested in a cell based assay, and was found to be about the same as commercial IL-2 on a per mole basis, to within the accuracy of the assay. The biological activity of the IL-2 moiety was tested in the CTLL-2 cell proliferation assay, as described in Example 4. The results indicate that murine IL-2, murine scIL-12-IL-2, and murine Fc-IL-12-IL-2 fusion protein were about equally potent in stimulating proliferation., Murine IL-12 causes no detectable stimulation of CTLL-2 cell proliferation. These results indicate that the stimulation of CTLL-2 cell proliferation by scIL-12-IL-2 fusion proteins was due to the IL-2 moiety and not the IL-12 moiety.

The IL-12 and IL-2 activities of the Fc-IL12-IL2, IL12-KS-IL2, and IL12-KS(Light chain) + KS(Heavy chain)-IL2 proteins described in Example 5 were also tested in cell-based assays. Using the PBMC cell proliferation/tritiated thymidine incorporation assay, the Fc-IL12-IL2, IL12-KS-IL2, and IL 12-KS(Light chain) + KS(Heavy chain)-IL2 proteins all showed potent IL-12 activity. Similarly, using the CTLL-2 cell proliferation assay, the Fc-IL12-IL2, IL12-KS-IL2, and IL12-KS(Light chain) + KS(Heavy chain)-IL2 proteins all showed potent IL-2 activity. In addition, in an ELISA, the IL12-KS-IL2 and IL12-KS(Light chain) + KS(Heavy chain)-IL2 proteins both bound tightly to the EpCAM antigen, even though the heavy and light chain V regions, respectively, are fused to other proteins at their N-termini.

### Example 8. Treatment of colon carcinoma in an immune-proficient mammal with a multiple cytokine fusion protein.

To test whether a multiple cytokine-antibody fusion protein could be used to treat colon carcinoma in a mammal with an intact immune system, the following experiments were performed. CT26 is a colon carcinoma cell line derived from Balb/C mice. By standard genetic engineering techniques, this cell line was engineered to express the human epithelial cell adhesion molecule (EpCAM), which is the antigen recognized by the KS-1/4 antibody; these cells are termed CT26/KSA cells.

Balb/C mice were subcutaneously inoculated with 2x10⁶ CT26/KSA cells. When tumors reached a volume of about 100-200 cubic millimeters, mice were randomized into three groups of 9 mice for further study. Beginning at day 0, tumor-bearing mice were treated with PBS, about 3.4 micrograms of KS-IL2 mixed with about 5.3 micrograms of KS-IL12, or about 6 micrograms of KS-IL2-IL12. These doses are designed to deliver an equal number of IL-12 and IL-2 molecules to each set of mice. Mice were injected intratumorally, once per day for five days. Tumor sizes were measured with calipers.

The results of one such experiment are shown in Figure 11. In this experiment, KS-IL12-IL2 caused a profound inhibition of tumor growth. The mixture of KS-IL12 and KS-IL2 also caused a significant inhibition of tumor growth, but not as complete as KS-IL12-IL2. In the group of mice treated with KS-IL12-IL2, six of nine mice were apparently cured of their tumors: these six mice survived until day 93, when the experiment was terminated; and the tumors in these mice shrank and disappeared, so that no subcutaneous tumor could be detected from day 39 to day 93. The other three mice had tumors whose growth was delayed such that the tumor volumes exceeded 4,000 cubic millimeters only after day 87.

Of the mice treated with a mixture of KS-IL12 and KS-IL2, two mice were apparently cured of their subcutaneous tumors and survived until the end of the experiment. The tumors in the remaining seven mice did not disappear and eventually grew to volumes of 1,000 cubic millimeter (1 mouse) or greater than 4,000 cubic millimeters (6 mice).

The fact that KS-IL12-IL2 is more effective that an equimolar mixture of KS-IL12 and KS-IL2 is surprising. The doses in this experiment deliver about 15 picomoles of fusion protein per dose, which corresponds to about 9x10¹² molecules. At the start of treatment, each tumor has a volume of about 160 cubic millimeters, which corresponds to about 160 million cells. Each cell expresses about 10⁶ molecules of EpCAM, so there are about 1.6x10¹⁴ EpCAM antigen molecules to which the KS antibody might bind. Thus, when KS-IL12 and KS-IL2 were mixed and injected into mice bearing such tumors, it is unlikely that these two immunocytokine fusion proteins competed with each other for antigen binding sites. Thus, the effective dose of IL-12 and IL-2 at the tumor site should have been at least as high for the mixture of KS-IL12 and KS-IL2 as for KS-IL12-IL2.

### Example 9. Treatment of colon carcinoma in an immunodeficient mammal with a multiple cytokine fusion protein.

Many forms of cancer therapy have the effect of killing dividing cells, including cells of the immune system. As a result, cancer patients often become immunosuppressed. To address whether multiple cytokine fusion proteins can be used to treat a mammal with a suppressed immune system, SCID mice bearing CT26/KSA tumors were treated with KS-IL12-IL2, a mixture of KS-IL12 and KS-IL2, or PBS. SCID mice are deficient in both B cells and T cells and depend on branches of the innate immune system, such as NK cells, for their ability to fight infections.

Mice with subcutaneous CT26/KSA tumors were generated as described in Example 8. Three groups of 8 mice each, bearing tumors of about 100 to 200 cubic millimeters, were treated by intratumoral injection with the same dosing and schedule as in Example 8. Results are shown in Figure 12. In this case, the KS-IL12-IL2 fusion protein and the mixture of KS-IL12 and KS-IL2 were about equally effective: five of eight mice were cured in each group by day 25. However, in the mice that were not cured, five of six tumors began to grow at a rate characteristic of tumors in untreated animals, with an effective delay of about 14 to 21 days. This is in contrast to tumors in immune-proficient mice in Example 8: even when tumors were not completely eliminated by treatment with KS-IL12-IL2, the tumors did not begin to grow aggressively until about 60 days after the beginning of the experiment.

These experiments demonstrate that a multiple-cytokine antibody fusion protein can be used to treat cancer in an immunosuppressed animal.

### Example 10. Treatment of lung carcinoma by intratumoral injection of a multiple cytokine fusion protein: comparison with treatment by incrividual immunocytokines

To address the effectiveness of multiple cytokine fusion proteins and immunocytokines carrying single cytokine moieties against a lung call-derived cancer, the following experiment was performed.

Lewis Lung Carcinoma (LLC) is an aggressive tumor derived from C57BU6 mice. An LLC cell line expressing the human EpCAM protein was constructed by standard genetic engineering techniques; the cell line was termed LLC/KSA.

C57BL/6 mice with subcutaneous LLC/KSA tumors were generated as described in Example 8 (check # of cells with KML). Four groups of 5 mice each, bearing tumors of about 100 to 200 cubic millimeters, were treated by intratumoral injection for five days. Mice were injected with PBS, about 20 micrograms of KS-IL12, about 20 micrograms of KS-IL12, or about 20 micrograms of KS-IL12-IL2.

Results are shown in Figure 13. In this case, the KS-IL12-IL2 fusion protein was much more effective than either KS-IL12 or KS-IL2- In all of the mice treated with the KS-1L12-IL2 fusion protein, the tumors disappeared by day 27. On day 74, these mice were used in a lung metastasis assay as described in Example 14; the original subcutaneous tumors did not reappear in the intervening period or during the second experiment. In contrast, treatment with either KS-IL2 or KS-IL12 resulted in some apparent tumor shrinkage and a significant delay in tumor growth, but the tumors did eventually grow. A comparison of the results in this example and previous examples indicates that, for certain diseases and modes of administration, treatment with a mixture of immunocytokines carrying different cytokine moieties is superior to treatment with a single type of immunocytokine.

### Example 12. Treatment of lung carcinoma by intratumoral injection of a multiple cytokine fusion protein: comparison with treatment by a mixture of immunocytokines

To address the effectiveness of multiple cytokine fusion proteins and mixtures of immunocytokines carrying different cytokine moieties against a lung cell-derived cancer, the following experiment was performed.

C578L/6 mice with subcutaneous LLC/KSA tumors were generated as described in Example 11. Three groups of 7 mice each, bearing tumors of about 100 to 200 cubic millimeters, were treated by intratumoral injection for five days. Mice were injected with PBS, a mixture of about 18 micrograms of KS-IL12 and about 11.5 micrograms of KS-IL12, or about 20 micrograms of KS-IL12-IL2.

Results are shown in Figure 14. In this case, the KS-IL 12-IL2 fusion protein was much more effective than the mixture of KS-IL12 and KS-IL2. In all of the mice treated with the KS-IL12-IL2 fusion protein, the tumors disappeared by day 27. In contrast, treatment with the mixture of KS-IL12 and KS-IL2 resulted in some apparent tumor shrinkage and a significant delay in tumor growth, but all of the tumors in this treatment group did eventually regrow.

### Example 13. Antigen-dependence of anti-tumor activity of a multiple cytokine-antibody fusion protein

To address the whether the effectiveness of a multiple cytokine-antibody fusion protein in treatment of a tumor was dependent on the tumor-specific expression of the antigen recognized by the antibody, the following experiment was performed.

A set of seven C57BU6 mice with subcutaneous LLC/KSA tumors and a second set of nine mice with tumors derived from the parental LLC cell line were generated as described in Example 11. These two groups of mice, bearing tumors of about 100 to 200 cubic millimeters, were treated by intratumoral injection for five days. Mice were injected with about 20 micrograms of KS-IL12-IL2.

Results are shown in Figure 15. In this case, the mice bearing the LLC/KSA tumors all were completely cured of their tumors. In contrast, only two of the mice bearing the LLC tumors were cured; the other LLC tumor-bearing mice all enjoyed a transient reduction in their tumor volumes, but their tumors eventually grew to large volumes.

These results indicate that the recognition of the EpCAM surface antigen promotes the adherence of KS-1L12-IL2 to the surface of LLC/KSA tumor cells, and the resulting immune response is enhanced. Some anti-tumor effect was observed against LLC-derived tumors as well; without wishing to be bound by theory, the antitumor effect of KS-IL12-IL2 in this case may be due to the fact that the fusion protein was injected directly into the tumor and was therefore transiently localized to the tumor.

### Example 14. Generation of an immune memory against a tumor cell type

The development of metastases is a major problem in treatment of cancer. To test whether treatment with a multiple cytokine antibody fusion protein could lead to formation of a long-lasting immune memory against a tumor cell type and could prevent the establishment of metastases, the following experiment was performed.

Five C57BU6 mice from Example 12 had been treated with KS-IL12-IL2, and had apparently been cured of their subcutaneous tumors. On day 74 relative to the initiation of treatment as described in Example 14, these five mice were injected i. v. with 10⁶ LLC/KSA cells. As a control, eight C57BU6 mice were also injected i. v. with 10⁶ LLC/KSA cells.

On day 28, the mice were sacrificed and the lungs were examined for metastases. The lungs of the eight control mice were 70% to 100% covered with metastases, with an average of 85% lung surface coverage. The mean lung weight for these mice was 0.86 grams. In contrast, there were no metastases found on the surface of lungs from the five pre-treated mice, and the average lung weight was 0.28 grams, which corresponds to the weight of a normal mouse lung. These results indicated that the treatment of the original tumor cells resulted in a long-lasting immune memory against the tumor cells; this memory prevented the establishment of metastases of this tumor cell type.

**Table X. Protection of "regressed" mice from LLC-KSA pulmonary metastases**

| Prior Treatment | Metastatic Score | Lung Weight (g) |
|---|---|---|
| None | 4, 4, 4, 4, 4, 4, 3, 3 | 0.88 +/- 0.27 |
| KS-IL12/IL2 | 0, 0, 0, 0, 0 | 0.27 +/- 0.03 |

The average lung weight of the control group, without tumor, was 0.2 g. Metastatic scores are based on % surface coverage of fused metastatic nodules where 0 = no metastases; 1 = 1-25% coverage; 2 = 25-50% coverage; 3 = 50-75% coverage; and 4 = 75-100% coverage

A second experiment to test for immune memory formation used six of the seven mice from Example 12 that had been injected with LLC/KSA tumor cells, had developed subcutaneous tumors, and had had those tumors disappear. Sixty-two days after the initiation of the treatment in Example 12, six pre-treated mice and 10 naïve, untreated C57BU6 control mice were injected s. c. with 10⁶ LLC cells. These cells do not express the human KS antigen, EpCAM.

In the naïve mice, the injected LLC cells formed tumors that grew at a rapid rate in all mice. In contrast, the tumors in the pre-treated mice grew much more slowly, and in one mouse, no subcutaneous tumor was detected. The results are shown in Figure 16.
Because the human KS antigen, EpCAM, is not expressed on LLC cells, the immune response to the LLC cells was based on other antigens expressed by these cells.

### Example 15: Multiple cytokine fusion proteins as vaccines

Multiple cytokine fusion proteins may be used as vaccines when fused to an antigen protein. The particular order of moieties from N-terminus to C-torminus, or whether the fusion protein is a single polypeptide chain or an oligomer, may vary depending on convenience of construction of the expressing plasmids. The protein may be administered by a variety of routes, such as intravenous, subcutaneous, intraperitoneal, and so on. Similarty, the dose and frequency of administration generally need to be empirically determined, as is standard practice for human vaccines and as is well known to those skilled in the art of vaccine development.

For example, a fusion protein of the form antigen-IL-12-cytokine is administered to a mouse, where the cytokine in the fusion protein is a second cytokine different from IL-12. Control mice receive the same amount of antigen-cytokine, antigen-IL-12, or antigen alone. At various times during and/or after administration of the antigen fusion protein, blood samples are collected by retro-orbital bleeding and plasma is prepared and analyzed for the presence of antibodies directed against the antigen. It is found that antibodies are generated against the antigen. Moreover, the nature of the immune response to the antigen is characteristic of a Th1 response. The antibody response is stronger and the type of antibodies produced are different than in certain control immunizations.

More specifically, a humanized antibody-murine IL-12-IL-2 fusion protein in PBS buffer, is injected into Balb/c mice intravenously (5 µg/day x 5). Control mice receive the same antibody, in the same amounts, but with no attached IL-12-IL-2. Neither injection solution contains any other type of adjuvant On day 10, blood samples are collected into microcentrifuge tubes by retro-orbital bleeding and plasma is prepared by collecting blood samples in plastic tubes containing sodium citrate, followed by centrifugation at full speed in an Eppendorf tabletop microcentrifuge. ELISA plates (96-well) are coated with the humanized antibody protein, which contains the human constant region and is used to capture any mouse antibodies made in response to the immunization. After washing away unbound material, the bound mouse antibodies are detected with goat anti-mouse Fc antibody (Jackson ImmunoResearch) coupled to horse-radish peroxidase. Any bound antibodies could be directed to either the human constant regions or the variable region, both of which are shared between the humanized antibody and the fusion proteins.

There is little or no reactivity to the humanized antibody without fused IL-12-IL-2. The fusion protein, on the other hand, induces a strong antibody response in the absence of exogenous adjuvants and despite the fact that the intravenous route of administration is highly unfavorable for inducing such responses, compared to either subcutaneous or intraperitoneal administration. Antibodies of the IgG2a isotype, which are typical of IL-12-enhanced responses, are seen in the antibody-IL-12-IL-2 injected group but not the group injected with the humanized antibody.

The immunogenicity of antigen-IL-12 multiple cytokine fusion proteins administered by various routes is tested by injecting a solution of the fusion protein (such as that described above) in PBS or other biocompatible buffer, or a known adjuvant such as Freund's incomplete or complete adjuvant. For example, single or multiple subcutaneous, intradermal or intraperitoneal injections can be given every two weeks. Alternatively, the fusion protein can be administered first by subcutaneous injection and then followed by intraperitoneal injection. Freund's adjuvant cannot be used for human use, due to the irritation at the injection site. Alternative adjuvants such as precipitates of aluminum hydroxide (Alum) are approved for human use and can be used in the present invention. New organic chemical adjuvants based on squalenes and lipids can also be used for injections into the skin.

### Example 96; Gene therapy with multiple cytokine fusion proteins

The anti-cancer activity of multiple cytokine fusion proteins delivered by gene therapy methods was also demonstrated for treatment of lung cancer. Lewis Lung Carcinoma cells were stably transfected using the viral vector system described above (pLNCX-scIL-12-IL-2 or pLNCX-scIL-12 DNA transfected into the PA317 packaging cell line). These constructs encode a single-chain version of IL-12, in which the p35 and p40 subunits have been connected with a linker. Clones were selected in vitro using G418-containing medium, and clones stably expressing about 50 to 60 ng/ml of IL-12 were identified by ELISA (R & D Systems).

About 1x10⁶ and about 5x10⁶ LLC cells expressing sclL-12 or sclL-12-IL-2 were injected s.c. into C57BU6 mice and also into SCID mice. As a control, 2x10⁶ LLC cells were injected into C57BL/6 mice and also into SCID mice. The LLC cells expressing IL-12 form tumors that grow at about the same rate as tumors derived from LLC cells that have not been engineered to express cytokines. However, in both C57BU6 mice and also in SCID mice, LLC cells expressing sclL-12-IL-2 either did not form subcutaneous tumors or formed tumors that subsequently shrank and disappeared (Figures 17 and 18).

### Example 18. Construction of DNA encoding lymphotactin-KS-IL2 and expression of the lymphotactin-KS-IL2 protein.

Chemokines are a distinct class of cytokines that are thought to form gradients and mediate chemotaxis of immune cells. In addition, like other cytokines, chemokines may induce expression of specific genes in target cells. One characteristic of chemokines is that the free N-terminus is often required for activity, which could place limits on the ways that fusion proteins could be constructed.

A cytokine-antibody-cytokine fusion protein was constructed consisting of the cytokine lymphotactin, which is a chemokine, the antibody KS-1/4, and the cytokine IL-2. This fusion protein was tetrameric and comprised two different polypeptides. One polypeptide consisted of murine lymphotactin fused to the N-terminus of the heavy chain of the KS-1/4 antibody, followed by IL-2 at the C-terminus. The fusion of the KS-1/4 heavy chain with IL-2 at the C-terminus, the "KS-IL2 heavy chain," has been described previously [Gillies et al. (1992) Proc. Natl. Acad. Sci. USA 89:1428]. The other polypeptide consisted of the light chain of the KS1/4 antibody.

The complete coding sequence of murine lymphotactin was published by Kelner and Zlotnik (Science 266:1395 [1998]). To construct DNA encoding a fusion protein of murine lymphotactin and the heavy chain of the KS-IL2, the murine lymphotactin cDNA was adapted by PCR using the forward primer *TCTAGA*GCCACC **ATG** AGA CTT CTC CTC CTG AC (SEQ ID NO:29), in which an XbaI site *TCTAGA* (residues 1-6 of SEQ ID NO:29) was placed upstream of the translation initiation codon **ATG**, and the reverse primer *GGA TCC* **CCC** AGT CAG GGT TAC TGC TG (SEQ ID NO:30), which placed a BamHI site *GGA TCC* (residues 1-6 of SEQ ID NO:30) immediately 3' of the GGG codon (anticodon CCC) encoding the C-terminal amino acid residue of murine lymphotactin. After cloning of the PCR fragment and sequence verification, the XbaI-BamHI fragment containing the murine lymphotactin cDNA was ligated to an BamHI-AflII oligonucleotide duplex encoding a flexible peptide linker rich in glycine and serine residues. The AfIII end was in turn joined to an artificial AfIII site preceding the nature N-terminus of the KS-IL2 heavy chain. The DNA sequence at the junctions resulting from the two ligations is given below: *AGC* **CAG** (SEQ ID NO:31)
where *GGATCC* (residues 4-9 of SEQ ID NO:31) and *CTTAAG* (residues 48-53 of SEQ ID NO: 31) are the two restriction sites BamHI and AflII, respectively, used for reconstruction; **CCC** encodes the C-terminal amino acid residue of murine lymphotactin; **CAG** encodes the mature N-terminus of the KS-IL2 heavy chain; and the amino acid sequence of the GlySer-rich peptide linker is shown above the DNA sequence. The DNA encoding the murine lymphotactin-KS-IL2 heavy chain was then cloned into an expression vector and then coexpressed with the KS1/4 light chain.

The expressed lymphotactin-KS-Il2 fusion protein is tested for lymphotactin activity in a Boyden chamber migration assay using T cells (Leonard et al., [1999] Current Protocols in Immunology p. 6.12.3). Alternatively, NK cells are used. Alternatively, lymphotactin activity is observed in a standard cellular assay for calcium flux in response to the activation of a G-protein coupled receptor (Maghazachi et al., FASEB J. [1997];11:765-74.). In addition, the tymphotactin-KS-IL2 fusion protein is tested and found to be active in assays for the ability to bind to EpCAM and is also active in assays for IL-2 activity, such as the CTLL-2 cell proliferation assay.

## Claims

1. A cytokine-antibody fusion protein having the structure KS-IL12-IL2, wherein KS is antibody KS-1/4.

2. A nucleic acid encoding a fusion protein of claim 1.

3. A pharmaceutical composition comprising a fusion protein of claim 1, optionally together with a pharmaceutical carrier or excipient.

4. A fusion protein of claim 1 for use for the treatment of colon or lung cancer.

## Patentansprüche

1. Cytokin-Antikörper-Fusionsprotein mit der Struktur KS-IL12-IL2, wobei KS für den Antikörper KS-1/4 steht.

2. Nukleinsäure, die ein Fusionsprotein nach Anspruch 1 kodiert.

3. Pharmazeutische Zusammensetzung, die ein Fusionsprotein nach Anspruch 1, gegebenenfalls zusammen mit einem pharmazeutischen Träger oder Exzipienten, umfasst.

4. Fusionsprotein nach Anspruch 1 für die Verwendung zur Behandlung von Colon- oder Lungenkrebs.

## Revendications

1. Protéine de fusion d'anticorps de cytokine présentant la structure KS-IL12-IL2, dans laquelle KS est KS-1/4 d'anticorps.

2. Acide nucléique codant une protéine de fusion selon la revendication 1.

3. Composition pharmaceutique comprenant une protéine de fusion selon la revendication 1, en option en association avec un vecteur ou excipient pharmaceutique.

4. Protéine de fusion selon la revendication 1 pour une utilisation pour le traitement du cancer du côlon ou du poumon.
